# EUROPÄISCHE PATENTSCHRIFT

(11) **EP 2 088 942 B1**
(45) Veröffentlichungstag und Bekanntmachung des Hinweises auf die Patenterteilung: **15.01.2014**
(21) Anmeldenummer: 07819787.8
(22) Anmeldetag: 13.11.2007
(51) Int. Cl.: A61B 17/32, A61M 5/142

(54) **MEDIZINISCHE PUMPE**
MEDICAL PUMP
POMPE MÉDICALE

(30) Priorität: 14.11.2006 DE 102006053609
(43) Veröffentlichungstag der Anmeldung: 19.08.2009
(73) Patentinhaber: ERBE Elektromedizin GmbH, 72072 Tübingen (DE)
(72) Erfinder: KÜHNER, Ralf, 70567 Stuttgart (DE); MITZLAFF, Lothar, 8600-531 Lagos (PT); WAHL, Hans-Jürgen, 72818 Trochtelfingen (DE); BAUER, Steffen, 71154 Nufringen (DE)
(74) Vertreter: Bohnenberger, Johannes
(86) Internationale Anmeldenummer: PCT/EP2007/009807
(87) Internationale Veröffentlichungsnummer: WO 2008/058704

(56) Entgegenhaltungen:
- EP-A- 1 484 071
- EP-A- 1 588 729
- WO-A-01/37905
- WO-A-2006/002815

## Beschreibung

Die Erfindung betrifft eine medizinische Pumpe, insbesondere für die Wasserstrahlchirurgie.

In der Leberchirurgie wird seit einiger Zeit die Wasserstrahlchirurgie genutzt, da dieses Organ wie kein anderes über Gewebestrukturen unterschiedlicher Festigkeit verfügt (Parenchym, Blutgefäße, Gallengänge) und somit der applizierte Wasserstrahl das zu schneidende Gewebe (Parenchym) zwar durchtrennt, die Blutgefäße und Gallengänge jedoch unbeschädigt lässt. Hierzu ist natürlich eine exakte Steuerung des Schneidstrahls und auch des Schneiddruckes notwendig.

Ferner ist in der Wasserstrahlchirurgie darauf zu achten, dass eine absolute Sterilität des Schneidmediums (z.B. Ringer-Lösung) gegeben sein muss, da die Flüssigkeit in die denkbar engste und intensivste Verbindung mit dem Körpergewebe gelangt. Um eine absolute Sterilität zu gewährleisten, sollten z. B. auswechselbare Teile auf einfachste Weise ausgetauscht werden können. Darüber hinaus sind natürlich die üblichen Probleme bzgl. hoher Zuverlässigkeit, Einfachheit und kostengünstiger Herstellbarkeit zu beachten.

Aus der US 6,216,573 B1 und aus der DE 203 09 616 U1 sind medizinische Pumpen für die Wasserstrahlchirurgie bekannt, die auswechselbare, also zur einmaligen Verwendung ausgebildete Pumpeneinheiten aufweisen, die mit Pumpenbetätigungseinrichtungen verbindbar sind. Das Auswechseln der Pumpeinrichtungen bzw. der Pumpeneinheiten ist jedoch bei den bekannten Anordnungen sehr aufwändig. Dadurch nämlich, dass relativ große Kräfte benötigt werden, um einen hohen Druck bei hinreichenden Durchflussmengen zu erzeugen, müssen die Einrichtungen zum Verbinden der Pumpeinrichtung mit den Pumpenbetätigungseinrichtungen sehr stabil ausgebildet sein und die Pumpeinrichtung "fest im Griff" haben.

Weitere medizinische Pumpen sind aus der WO 01/37905 A2 und aus dem Stand der Technik nach WO 2006/002815 A1, im Oberbegriff von Patentanspruch 1 gewürdigt, bekannt.

Der Erfindung liegt die Aufgabe zu Grunde, eine medizinische Pumpe der eingangs genannten Art dahin gehend weiter zu bilden, dass die Verbindung zwischen der Pumpeneinheit bzw. Pumpeinheit und der Pumpenbetätigungseinrichtung verbessert und ihre Bedienbarkeit erleichtert wird.

Diese Aufgabe wird durch eine medizinische Pumpe nach dem Patentanspruch 1 gelöst. Insbesondere wird die Aufgabe durch eine medizinische Pumpe, insbesondere für die Wasserstrahlchirurgie gelöst, die Folgendes umfasst:
- eine Pumpeneinheit mit zwei Kolben zum Verschieben der Kolben in zugeordneten Zylindern;
- eine Pumpenbetätigungseinrichtung zum alternierenden Verschieben der Kolben, umfassend
   - eine öffen- und schließbare Halteeinrichtung zum Befestigen und Lösen der Pumpeneinheit an der oder von der Pumpenbetätigungseinrichtung;
   - öffen- und schließbare Kupplungseinrichtungen zum Ankoppeln oder Entkoppeln der Kolben an die oder von der Pumpenbetätigungseinrichtung;
   - mindestens eine Antriebseinrichtung und eine Steuerung zum Betätigen der Pumpeneinheit durch Verschieben der Kolben,
      wobei die Antriebseinrichtung zum Betätigen, insbesondere zum Öffnen und/oder Schließen, der Halteeinrichtung und/oder der Kupplungseinrichtungen derart steuerbar ausgebildet ist, dass ein Arbeits- oder ein Ruhezustand gebildet wird; und wobei die Antriebseinrichtung mit zwei Motoren oder einem Motor mit Getriebe derart steuerbar ausgebildet ist, dass die Kolben in einer vom alternierenden Betrieb abweichenden Weise zum Zweck des Öffnens oder Schließens der Halteeinrichtung und/oder der Kupplungseinrichtungen betrieben werden.

Die medizinische Pumpe zeichnet sich dadurch aus, dass
- ein Detektionsmittel vorgesehen ist, das mit einem Eingang der Steuerung kommunikativ verbunden und derart ausgebildet ist, dass es einen eine vorbestimmte Lagebeziehung zwischen Pumpeneinheit und Pumpenbetätigungseinrichtung definierenden Einsetzzustand detektiert und ein erstes, die Anwesenheit der Pumpeneinheit in der Pumpenbetätigungseinrichtung bezeichnendes Detektionssignal an die Steuerung zum Steuern der Antriebseinrichtung übermittelt, und wobei das Detektionsmittel derart ausgebildet ist, dass es ein zweites, die Nicht-Anwesenheit der Pumpeneinheit in der Pumpenbetätigungseinrichtung bezeichnendes Detektionssignal an die Steuerung zum Steuern der Antriebseinrichtung übermittelt;
wobei die Steuerung zur Verarbeitung des ersten Detektionssignals derart ausgebildet ist, dass sie die Antriebseinrichtung zur Bildung des Arbeitszustandes steuert, und wobei die Steuerung zur Verarbeitung des zweiten Detektionssignals derart ausgebildet ist, dass sie die Antriebseinrichtung zur Bildung des Ruhezustandes steuert.

Ein wesentlicher Punkt der Erfindung liegt also darin, dass die Antriebseinrichtung, die an sich zum Hin- und Herschieben der Kolben vorgesehen ist, zusätzlich auch die Halteeinrichtungen und/oder die Kupplungseinrichtungen betätigt bzw. steuert und das Befestigen und Lösen der Pumpeneinheit an der oder von der Pumpenbetätigungseinrichtung bzw. das Ankoppeln oder Entkoppeln der Kolben an die oder von der Pumpenbetätigungseinrichtung zumindest teilweise aufgrund der Detektion der Lagebeziehung zwischen Pumpeneinheit und Pumpenbetätigungseinrichtung ausgelöst werden. Dabei muss ein Bediener die Pumpeneinheit mir nur geringem Kraftaufwand in die Halteeinrichtung einsetzen, so dass sie bereits durch die Halteeinrichtung mindestens teilweise festgehalten wird. Die (ggf. vollständige) Befestigung bzw. die Ankopplung an der Pumpenbetätigungseinrichtung erfolgen dann durch das System selbsttätig, es wird also selbsttätig ein Arbeitszustand ausgebildet. Dazu detektieren die Detektionsmittel einen die vorbestimmte Lagebeziehung zwischen Pumpeneinheit und Pumpenbetätigungseinrichtung definierenden Einsetzzustand (Pumpeneinheit in Pumpenbetätigungseinrichtung eingesetzt oder nicht) und übermitteln ein entsprechendes Signal an die Steuerung (in der Regel dann, wenn die Pumpeneinheit in die Halteeinrichtung eingesetzt ist). Diese steuert die Antriebseinrichtung derart an, dass die Befestigungs- und Ankopplungsvorgänge ausgeführt werden, sobald das Einsetzen angezeigt wird. Auf diese Weise ist eine einfache und sichere Bedienbarkeit der medizinischen Pumpe gewährleistet. Auch die Entkopplung bzw. das Lösen der Pumpeneinheit von Pumpenbetätigungseinrichtung kann detektiert werden, so dass das System in eine Art Ruhezustand überführt wird.

Arbeitszustand bedeutet, dass die Pumpeneinheit derart mit der Pumpenbetätigungseinrichtung verbunden ist, dass ein Betriebszustand erreicht ist, der das System (d. h. die medizinische Pumpe) für die Bedienperson - ohne dass diese noch weitere Schritte ausführen müsste - einsatzbereit macht. Der Arbeitszustand ist dann gegeben, wenn die Pumpeneinheit in der Halteeinrichtung befestigt und die Kolben in dem Kupplungssystem angekoppelt sind. Ruhezustand meint hier, dass das System einen Zustand erreicht hat, der das Einsetzen einer neuen Pumpeneinheit ermöglicht.

Vorzugsweise ist mindestens ein Eingabeelement vorgesehen, das mit einem Eingang der Steuerung kommunikativ verbunden ist und ein Signal an die Steuerung übermittelt, wobei die Steuerung zur Verarbeitung des Signals derart ausgebildet ist, dass durch die Halteeinrichtung und/oder die Kupplungseinrichtungen eine Löse- und Entkoppelposition eingenommen wird.

Wie bereits oben angemerkt, wird der Einsetzzustand detektiert und daraufhin werden Befestigungs- und Ankopplungsvorgänge durchgeführt. Um nun ein Lösen und Entkoppeln der Pumpeneinheit bzw. der Kolben von der Pumpenbetätigungseinrichtung zu ermöglichen, muss der Steuerung ein entsprechendes Signal übermittelt werden. Dieses Signal wird durch das Betätigen des mit der Steuerung kommunikativ verbundenen Eingabeelements generiert. Die Steuerung ist in der Praxis mit einer Schalttafel oder einem Touchscreen ausgebildet, wobei entsprechende Taster oder Schalter oder auch entsprechende Bereiche auf einem Touchscreen vorgesehen sind, die das Anzeigen der gewollten Auflösung der Verbindung zwischen Pumpeneinheit und Pumpenbetätigungseinrichtung ermöglichen. Durch entsprechendes Betätigen eines Bereichs des Touchscreens (Eject-Taste), wird der Steuerung also angezeigt, dass die Pumpeneinheit aus der Pumpenbetätigungseinrichtung entfernt werden soll. Hierzu wird die Antriebseinrichtung derart angesteuert, dass sie das System in eine Löse- und Entkoppelposition verfährt, wobei in dieser Position bzw. spätestens bei Erreichen dieser Position dann das Entkoppeln der Kolben und das Lösen aus der Halteeinrichtung selbsttätig durch das System erfolgt (die Pumpeneinheit liegt dann entnahmebereit in der Pumpenbetätigungseinrichtung). Das endgültige Entfernen der Pumpeneinheit aus der Pumpenbetätigungseinrichtung kann dann durch die Bedienperson erfolgen.

Gemäß der Erfindung ist das Detektionsmittel derart ausgebildet, dass es ein zweites, die Nicht-Anwesenheit der Pumpeneinheit in der Pumpenbetätigungseinrichtung bezeichnendes Detektionssignal an die Steuerung zum Steuern der Antriebseinrichtung übermittelt, wobei die Steuerung zur Verarbeitung des zweiten Detektionssignals derart ausgebildet ist, dass sie die Antriebseinrichtung zur Bildung des Ruhezustandes steuert. Damit wird letztendlich das endgültige Entfernen der Pumpeneinheit aus der Pumpenbetätigungseinrichtung detektiert, so dass die Pumpenbetätigungseinrichtung in einen Zustand überführt wird, in dem eine neue Pumpeneinheit in die Pumpenbetätigungseinrichtung eingesetzt werden kann. Dieser Zustand ist im Prinzip der Ruhezustand.

Vorzugsweise ist das Detektionsmittel also derart ausgebildet, dass es das Detektionssignal generiert und an die Steuerung übermittelt, wenn die Pumpeneinheit in die Pumpenbetätigungseinrichtung eingesetzt und/oder aus dieser entfernt wird. Primär soll dann ein Signal (erstes Detektionssignal) generiert und an die Steuerung übermittelt werden, wenn die Pumpeneinheit in die Pumpenbetätigungseinrichtung, genauer in die Halteeinrichtung eingesetzt wird. Die Pumpeneinheit kann durch den Bediener derart in die Halteeinrichtung eingesetzt werden, dass sie sich zumindest in einem vorverriegelten Zustand befindet oder bereits vollständig in dieser befestigt ist. In jedem Fall wird durch das Einsetzen das Detektionsmittel "aktiviert", d. h., es erfasst den aktuellen Zustand der durch die Lagebeziehung zwischen Pumpeneinheit und Pumpenbetätigungseinrichtung gegeben ist. Durch das Einsetzen wird also erfasst, dass sich eine Pumpeneinheit in der Halteeinrichtung befindet (umgekehrt kann auch erfasst werden, dass keine Pumpeneinheit eingesetzt ist). Die Erfassung dieses Zustandes und die Generierung des Detektionssignals lösen dann das Befestigen und Ankoppeln aus. Das (ggf. vollständige) Befestigen und das Ankoppeln erfolgen gesteuert über den Antrieb, der eigentlich zum Betätigen der Pumpe selbst vorgesehen ist. Gesonderte Antriebseinrichtungen sind hierzu nicht notwendig. Im Übrigen erfolgt auch das Lösen und das Entkoppeln über den Antrieb.

Das Detektieren der Lagebeziehung zwischen Pumpeneinheit und Pumpenbetätigungseinrichtung ermöglicht ein präzises und sicheres Verbinden von Pumpeneinheit und Pumpenbetätigungseinrichtung zu einem vorbestimmten Zeitpunkt, nämlich dann, wenn die Pumpeneinheit in die Halteeinrichtung entsprechend eingesetzt wurde.

Eine Ausführungsform sieht vor, dass das Detektionsmittel eine Lichtschranke aufweist, wobei an der Pumpeneinheit eine Unterbrechereinrichtung zum Unterbrechen eines Lichtstrahles der Lichtschranke vorgesehen ist und wobei die Lichtschranke das erste Detektionssignal bereitstellt. Damit wird also auf einfache Weise die Detektion der Lagebeziehung und die Generierung des entsprechenden Signales zur Ansteuerung des Antriebs erreicht. Vorzugsweise ist die Lichtschranke als eine Gabellichtschranke ausgebildet, so dass die Unterbrechereinrichtung auf einfache Weise in die Gabel geschoben werden kann. Gabellichtschranken eignen sich für die präzise Erfassung kleinster Objekte und können auf kleinstem Bauraum untergebracht werden.

Vorzugsweise ist die Lichtschranke derart ausgebildet, dass sie auf Reflexion des von einem Sender der Lichtschranke ausgesendeten Lichtes anspricht. Dies ist insbesondere dann vorteilhaft, wenn die Unterbrechereinrichtung aus einem transluzenten Material ausgebildet ist und daher die Unterbrechung des Lichtstrahls durch die spezielle Ausgestaltung (z. B. durch eine Schräge) derart ermöglicht, dass sie den Lichtstrahl reflektiert und so ein entsprechendes Signal ausgelöst wird. Grundsätzlich kann die Unterbrechereinrichtung auch aus einem lichtundurchlässigen Material ausgebildet sein und so ein Auslösen der Lichtschranke durch Absorption bewirken.

Vorzugsweise arbeitet die Lichtschranke derart, dass ein "fehlendes" Signal am Empfänger die Lichtschranke auslöst. Möglich ist es natürlich auch, eine Lichtschranke vorzusehen, die einen Sender und einen Empfänger in getrennten Gehäusen untergebracht hat bzw. bei der Sender und Empfänger nebeneinander in einem Gehäuse platziert sind. Im Prinzip kann hier jede Art von Lichtschranke oder dergleichen Sensor eingesetzt werden.

In einer Ausführungsform weist das Detektionsmittel eine Taster- oder Schaltereinrichtung auf, wobei die Taster- oder Schaltereinrichtung das zweite Detektionssignal bereitstellt. Die Tastereinrichtung ist z. B. vorzugsweise als ein Mikroschalter oder -taster ausgebildet. Der Taster oder Schalter ist dann vorzugsweise derart an der Halteeinrichtung oder an einer anderen Komponente der Pumpenbetätigungseinrichtung angeordnet, dass er mit dem Einsetzen der Pumpeneinheit unproblematisch betätigt werden kann und sich beispielsweise solange in betätigtem Zustand befindet, solange die Pumpeneinheit eingesetzt ist. Nun kann durch Betätigung des Schalters oder Tasters das Signal generiert und an die Steuerung übermittelt werden, wie oben beschrieben. Möglich ist es jedoch auch, dass erst mit dem Loslassen des Tasters (ggf. auch mit der erneuten Betätigung eines Schalters) das Detektionssignal generiert wird, d. h. das Entfernen der Pumpeneinheit aus der Pumpenbetätigungseinrichtung wird angezeigt. Die Steuerung kann die Antriebseinrichtung dann derart ansteuern, dass die Pumpenbetätigungseinrichtung in einen vorbestimmten Zustand überführt wird, z. B. in den Ruhezustand, der das Einsetzen einer neuen Pumpeneinheit ermöglicht.

Bei Verwendung eines Tasters kann dieser solange betätigt bleiben, bis die Pumpeneinheit aus der Pumpenbetätigungseinrichtung entfernt wird. Dazu weist die Pumpeneinheit vorzugsweise eine Betätigungseinrichtung auf. Möglich ist es auch, einen Kippschalter vorzusehen, der während des Einsetzens und erneut während des Entfernens der Pumpeneinheit betätigt wird, so dass ein entsprechendes Signal an die Steuerung übermittelt wird.

Es ist also möglich, den Einsetzzustand mit nur einem Detektionsmittel zu erfassen und zwar sowohl das Einsetzen der Pumpeneinheit, als auch deren Entfernen. Das erste und das zweite Detektionssignal werden dann also von dem einen Detektionsmittel bereitgestellt. Es ist ferner aber auch möglich, für jeweils das erste und das zweite Detektionssignal getrennte Detektionsmittel (mindestens zwei) zur Verfügung zu stellen, so dass, wie oben beschrieben, die Lichtschranke das erste Detektionssignal und der Mikroschalter das zweite Detektionssignal bereitstellen.

In einer Ausführungsform umfassen die Pumpenbetätigungseinrichtung erste Schnappverbindungseinrichtungen und die Pumpeneinheit zweite Schnappverbindungseinrichtungen, so dass das Befestigen oder Lösen der Pumpeneinheit an der oder von der Pumpenbetätigungseinrichtung und/oder das Ankoppeln oder Entkoppeln der Kolben an die oder von der Pumpenbetätigungseinrichtung durch Ineinandergreifen der jeweiligen ersten und zweiten Schnappverbindungseinrichtungen oder ein voneinander Lösen erfolgt. Die Pumpeneinheit wird im Prinzip über zwei Kontaktbereiche mit der Pumpenbetätigungseinrichtung verbunden, nämlich der Halteeinrichtung und der Kupplungseinrichtungen. Halteeinrichtung und Kupplungseinrichtungen müssen derart ausgebildet sein, dass sie Gegenstücke der Pumpeneinheit aufnehmen und sicher verriegeln können. Nur so ist ein ordnungsgemäßer Pumpenbetrieb möglich.

Die ersten Schnappverbindungseinrichtungen der Pumpenbetätigungseinrichtung sind vorzugsweise als Achsbolzen der Halteeinrichtung und als Halteblöcke und zugehörige Federn der Kupplungseinrichtungen ausgebildet sind, während die zweiten Schnappverbindungseinrichtungen der Pumpeneinheit vorzugsweise als Haltestege an einem Ventildeckel und als Koppelvorsprünge an Kolbenenden ausgebildet sind. Sobald Pumpeneinheit und Pumpenbetätigungseinrichtung miteinander betriebsbereit verbunden sind, wirken die Achsbolzen mit den Haltestegen und die Halteblöcke und zugehörigen Federn mit den Koppelvorsprüngen zusammen, wenn Halteeinrichtung und Kupplungseinrichtungen geschlossen sind. Die Achsbolzen sind derart an der Halteeinrichtung angeordnet, dass sie, wenn die Pumpeneinheit in die Halteeinrichtung eingesetzt ist, sowohl an einer Unterseite der Pumpeneinheit als auch an einer Oberseite, nämlich an dem Ventildeckel mit jeweils einem Haltesteg in Eingriff kommen. Die Achsbolzen liegen also derart an den Haltestegen an, dass eine Entnahme der Pumpeneinheit nicht mehr möglich und die Pumpeneinheit sicher im Griff der Halteeinrichtung ist. Die Koppelvorsprünge der Kolben sind derart ausgebildet, dass sie zwischen Federenden der Federn in den Halteblöcken eingespannt und die Kolben so zug- und schubfest verklemmt sind. Damit ist die Pumpeneinheit sicher in der Pumpenbetätigungseinrichtung fixiert und die Pumpe kann in Betrieb genommen werden.

In einer anderen Ausführungsform sind die ersten Schnappverbindungseinrichtungen als Klauen an der Halteeinrichtung und als Halteblöcke und zugehörige Federn der Kupplungseinrichtungen ausgebildet. Die zweiten Schnappverbindungseinrichtungen sind als Haltevorsprünge an einem Zylinderkopf und als Koppelvorsprünge an Kolbenenden ausgebildet, wobei die Klauen mit den Haltevorsprüngen und die Halteblöcke und zugehörigen Federn mit den Koppelvorsprünge zusammenwirken, wenn Halteeinrichtung und Kupplungseinrichtungen geschlossen sind. Das Zusammenwirken ist im Prinzip ähnlich der oben beschriebenen Ausführungsform vorgesehen. Bei dieser Ausführungsform sind jedoch Klauen an der Halteeinrichtung angeordnet, die als klappbare Elemente in die Haltevorsprünge an der Pumpeneinheit eingreifen. Damit ist auch hier ein sicheres Befestigen der Pumpeneinheit an der Pumpenbetätigungseinrichtung gewährleistet.

Vorzugsweise ist die Steuerung derart ausgebildet, dass das Schließen der Halteinrichtung und/oder der Kupplungseinrichtungen durch ein mittels der Antriebseinrichtung durchgeführtes selbsttätiges Einschnappen der jeweiligen ersten und zweiten Schnappverbindungseinrichtungen und das Öffnen durch ein mittels der Antriebseinrichtung durchgeführtes selbsttätiges Öffnen der Schnappverbindungseinrichtungen durchführbar ist. Wie bereits oben beschrieben, sollen Befestigen, Ankoppeln, Entkoppeln und Lösen selbsttätig durch entsprechende Steuerung der Antriebseinrichtung durchführbar sein. Durch das Betätigen der Antriebseinrichtung und ein Verfahren der Halteblöcke über die Antriebseinrichtung können die Schnappverbindungseinrichtungen derart zueinander angeordnet und betätigt werden, dass das Einschnappen und deren Öffnen selbsttätig erfolgt. Es können große Haltekräfte genutzt werden, weil Öffnen und Schließen durch die Antriebseinrichtung durchgeführt wird.

Die Antriebseinrichtung umfasst vorzugsweise einen Linearantrieb (bzw. bei zwei Kolben zwei Linearantriebe) mit Spindel und Motor zum steuerbaren Antreiben der Spindel. Über derartige Linearantriebe können sehr exakte und darum die Pumpe mit ihren Kolben-/Zylindereinheiten schonende Bewegungen durchgeführt werden.

Gemäß der Erfindung sind zwei Kolben bzw. Kolbenstangen in Zylindern vorgesehen und die Pumpenbetätigungseinrichtung ist zum alternierenden Verschieben der Kolben ausgebildet. Dadurch kann eine erhöhte Pumpleistung sichergestellt werden. Bei dieser Ausführungsform ist die Antriebseinrichtung mit zwei Motoren oder einem Motor mit einem steuerbaren Getriebe vorzugsweise derart steuerbar ausgebildet, dass die Kolben in einer vom alternierenden Betrieb abweichenden Weise zum Zweck des Öffnens oder Schließens der Halteeinrichtung und/oder der Kupplungseinrichtungen betrieben werden. Während also beim normalen Pumpvorgang die Kolben alternierend betätigt werden, wird zum Öffnen oder Schließen der Halteeinrichtung und/oder der Kupplungseinrichtungen ein anderer Betriebsmodus mit ein und denselben Antriebsaggregaten gewählt, was einen vereinfachten Aufbau mit sich bringt.

Vorzugsweise ist die Motorsteuerung bzw. Steuerung derart ausgebildet ist, dass die Kolben in konstanter Geschwindigkeit verschiebbar sind. Dadurch ergibt sich eine gleichmäßigere Förderung des zu pumpenden Mediums.

Die Schnappverbindungseinrichtungen sind vorzugsweise derart ausgebildet, dass eine zum Schließen aufzubringende Kraft geringer als eine zum Öffnen aufzubringende Kraft ist, insbesondere die Halteeinrichtung betreffend. Dies ist insbesondere bei einer Ausführungsform vorteilhaft, bei der mit dem Einsetzen der Pumpeneinheit in die Halteeinrichtung bereits ein vollständiges Befestigen vorgesehen ist und nur noch das Ankoppeln der Kolben an das Kupplungssystem selbsttätig durch das Ansteuern der Antriebseinrichtung erfolgt. Die Bedienperson kann die Pumpeneinheit dann problemlos in die Halteeinrichtung einbringen. Gleichzeitig ist eine hohe Haltekraft erzeugbar, weil diese durch das selbsttätige Öffnen leicht überwunden werden kann.

Die Antriebseinrichtung und auch die Steuerung sind vorzugsweise derart ausgebildet, dass die in der Pumpenbetätigungseinrichtung angebrachten

Schnappverbindungseinrichtungen oder Rasteinrichtungen der Kupplungseinrichtungen in einem Ruhezustand vor Befestigen der Pumpeneinheit an der Pumpenbetätigungseinrichtung derart positioniert sind, dass bei einem Verbinden der Pumpeneinheit mit der Pumpenantriebseinrichtung die Rasteinrichtungen außer Eingriff mit den Kolbenstangen sind und die Kupplungseinrichtungen durch Betätigen der Antriebseinrichtungen schließbar sind. Dies bedeutet, dass der Benutzer keine größeren Manipulationen insbesondere in Bezug auf die Kolben bzw. Kolbenpositionen vornehmen muss, um die Pumpeneinheit an der Pumpenbetätigungseinrichtung anzubringen. Hier wird, wie bereits oben beschrieben, das Einsetzen der Pumpeneinheit in die Halteeinrichtung detektiert und die Antriebseinrichtung derart angesteuert, dass das Ankoppeln selbsttätig erfolgt.

Bevorzugte Ausführungsformen der Erfindung ergeben sich aus den Unteransprüchen.

Nachfolgend werden bevorzugte Ausführungsformen der Erfindung anhand von Abbildungen näher erläutert. Hierbei zeigen
- Fig. 1 ein schematisches Blockschaltbild einer Ausführungsform der medizinischen Pumpe,
- Fig. 2 eine perspektivische Explosionsdarstellung einer Ausführungsform der Pumpeneinheit,
- Fig. 3 eine perspektivische Darstellung der Pumpenbetätigungseinrichtung mit angekoppelter Pumpeneinheit,
- Fig. 4 eine Seitenansicht einer Halteeinrichtung,
- Fig. 5 eine Darstellung ähnlich der nach Fig. 4, jedoch in einem Teilschnitt entlang der Linie V-V aus Fig. 6,
- Fig. 6 eine Darstellung der Anordnung nach Fig. 4 in Draufsicht,
- Fig. 7-9 Darstellungen entsprechend denen nach den Fig. 4-6, jedoch in geöffnetem Zustand der Halteeinrichtung,
- Fig. 10 eine perspektivische Explosionsdarstellung von Funktionseinheiten der Halteeinrichtung,
- Fig. 11 eine perspektivische Darstellung einer Untereinheit aus Fig. 10,
- Fig. 12 eine Explosionsdarstellung einer Untereinheit aus Fig. 11,
- Fig. 13 eine perspektivische Darstellung von Kupplungseinrichtungen,
- Fig. 14 eine Darstellung der Kupplungseinrichtungen aus Fig. 13 in einer anderen Verschiebeposition,
- Fig. 15 eine Explosionsdarstellung einer Kupplungseinrichtung aus den Fig. 13 oder 14,
- Fig. 16 eine perspektivische Explosionsdarstellung einer Untereinheit der Anordnung nach Fig. 13,
- Fig. 17 eine Draufsicht der Anordnung nach Fig. 13 mit angekoppelten Kolbenstangen,
- Fig. 18 eine Vorderansicht der Anordnung nach Fig. 17,
- Fig. 19 eine Ansicht entsprechend der nach Fig. 17, jedoch in einem anderen Betriebszustand,
- Fig. 20 eine Vorderansicht auf die Anordnung nach Fig. 19,
- Fig. 21 einen Schnitt entlang der Linie XXI-XXI aus Fig. 19,
- Fig. 22 eine perspektivische Darstellung der Pumpenbetätigungseinrichtung mit noch nicht angekoppelter Pumpeneinheit in einer weiteren Ausführungsform,
- Fig. 23 einen Ausschnitt aus Fig. 22, vergrößert,
- Fig. 24 eine Draufsicht der Pumpenbetätigungseinrichtung gemäß Fig. 22,
- Fig. 25 einen Schnitt entlang der Linie XXV-XXV aus Fig. 24,
- Fig. 26 eine Draufsicht der Pumpeneinheit gemäß Fig. 22,
- Fig. 27 eine Seitenansicht der Pumpenbetätigungseinrichtung gemäß Fig. 22, Fig. 28 eine Seitenansicht der Pumpeneinheit gemäß Fig. 22,
- Fig. 29 eine perspektivische Darstellung der Pumpenbetätigungseinrichtung mit angekoppelter Pumpeneinheit im Betrieb,
- Fig. 30 eine Draufsicht der Pumpenbetätigungseinrichtung mit angekoppelter Pumpeneinheit gemäß Fig. 29,
- Fig. 31 einen Ausschnitt aus Fig. 30, vergrößert,
- Fig. 32 einen Schnitt entlang der Linie XXII-XXII aus Fig. 30,
- Fig. 33 eine Seitenansicht der Pumpenbetätigungseinrichtung mit angekoppelter Pumpeneinheit gemäß Fig. 29,
- Fig. 34 eine Draufsicht der Pumpenbetätigungseinrichtung mit angekoppelter Pumpeneinheit,
- Fig. 35 einen Schnitt entlang der Linie XXXV-XXXV aus Fig. 34,
- Fig. 36 eine Seitenansicht der Pumpenbetätigungseinrichtung mit angekoppelter Pumpeneinheit gemäß Fig. 34,
- Fig. 37 eine perspektivische Darstellung der Pumpenbetätigungseinrichtung,
- Fig. 38 eine vergrößerte Ansicht der Pumpenbetätigungseinrichtung mit angekoppelter Pumpeneinheit gemäß Fig. 24,
- Fig. 39 einen Schnitt entlang der Linie XXXIX-XXXIX aus Fig. 38,
- Fig. 40 eine vergrößerte Ansicht der Pumpenbetätigungseinrichtung mit
   angekoppelter Pumpeneinheit gemäß Fig. 30,
   Fig. 41 einen Schnitt entlang der Linie XLI-XLI aus Fig. 40,
- Fig. 42 eine Explosionsdarstellung einer geöffneten Halteeinrichtung,
- Fig. 43 eine perspektivische Ansicht eines Öffnungsschiebers,
- Fig. 44 eine perspektivische Darstellung der Pumpenbetätigungseinrichtung mit noch nicht angekoppelter Pumpeneinheit von unten.

In der nachfolgenden Beschreibung werden für gleiche und gleich wirkende Teile dieselben Bezugsziffern verwendet.

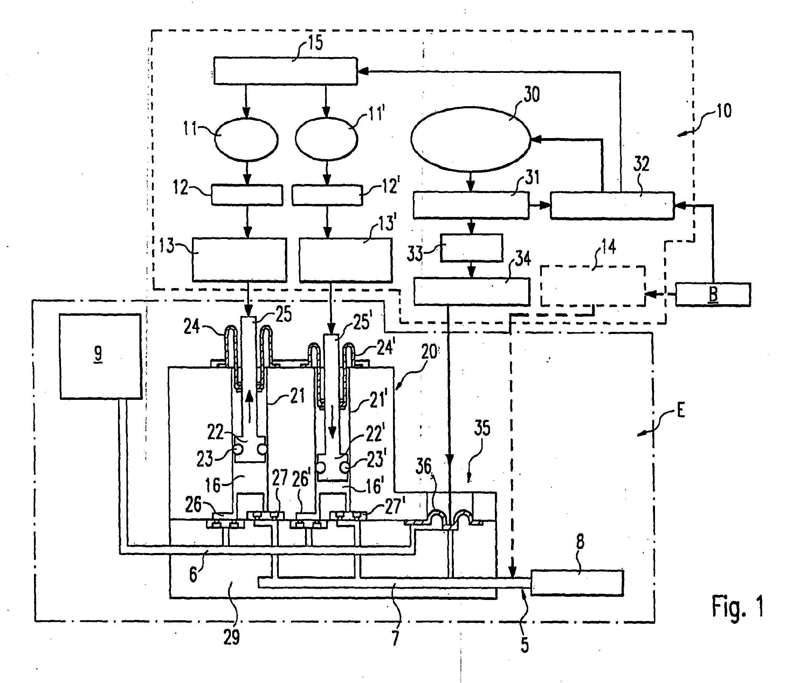

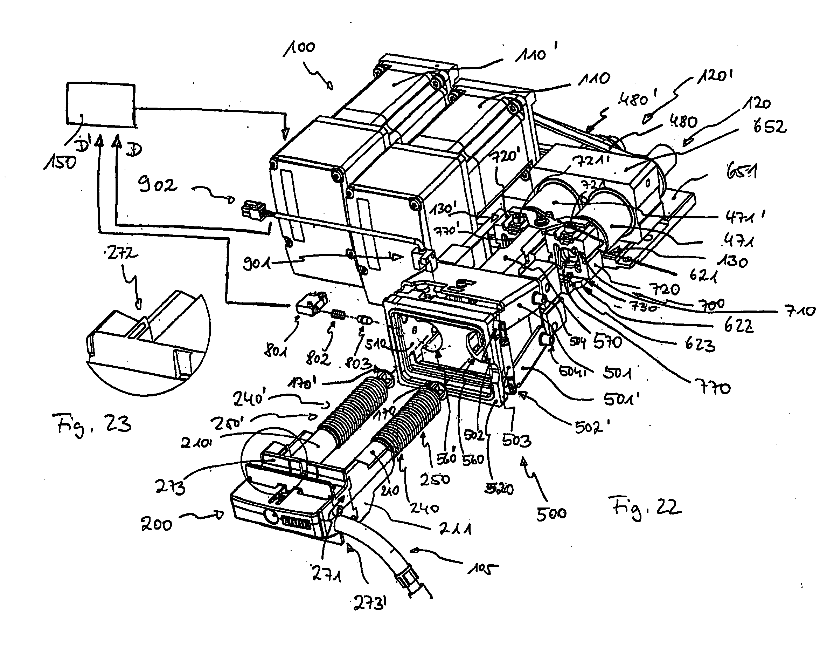

Im Prinzip zeigen die Fig. 1 bis 21 eine erste Ausführungsform der erfindungsgemäßen Pumpe, während die Fig. 22 bis 44 eine zweite, gegenüber der ersten Ausführungsform modifizierte Ausführungsform darstellen. Die Detektionsmittel, wie sie oben bereits angeführt wurden, sind nur in der zweiten Ausführungsform, also mit den Fig. 22 bis 44 gezeigt. Die in Rede stehende Detektionsmittel (oder Sensoreinrichtungen) sind jedoch auch in einem Pumpensystem, also einer medizinische Pumpe, wie es mit den Fig. 1 bis 21 aufgezeigt ist, vorgesehen, sie sind nur nicht explizit dargestellt. Fig. 1 lässt sich auf beide Ausführungsformen lesen, auch wenn dort nur die Bezugszeichen der ersten Ausführungsform angegeben sind.

Bei der in Figur 1 dargestellten Ausführungsform der Erfindung ist eine Pumpenbetätigungseinrichtung 10 vorgesehen, welche eine Motorsteuerung 15 zur Steuerung von zwei Motoren 11, 11' umfasst, die über Getriebe 12, 12' und Kupplungseinrichtungen 13, 13' mit Kolbenstangen 25, 25' verbunden sind. Eine Bedienungsperson B kann durch geeignete Schalteinrichtungen (Fußschalter, Fingerschalter) die Motorsteuerung 15 derart betätigen, dass die Motoren 11, 11' über die beschriebene Kette die Kolbenstangen 25, 25' und damit Kolben 22, 22' in Zylindern 21, 21' einer Pumpeneinheit 20 alternierend verschieben, so dass Druckräume 16, 16' der Pumpeneinheit 20 alternierend in ihrem Volumen vergrößert und verkleinert werden.

Zur Abdichtung der Druckräume 16, 16' bzw. der Kolben, 22, 22' gegenüber den Zylindern 21, 21' sind an den Kolben 22, 22' Dichtungen 23, 23' vorgesehen. Darüber hinaus sind die Kolbenstangen 25, 25' über Rollmembranen 24, 24', welche einerseits mit den Zylindern 21, 21' und andererseits mit den Kolbenstangen 25, 25' fest verbunden sind, keimdicht abgedichtet. Auf diese Weise können Keime, die sich aus der Umgebungsluft ohne diese Rollmembranen 24, 24' an den Innenwänden der Zylinder 21, 21' absetzen und von den Dichtungen 23, 23' durchgelassen werden, nicht mit dem Arbeitsfluid vermischen bzw. in dieses gelangen.

Bei der Ausführungsform gemäß der Fig. 22 bis 44 Faltenbalge statt der hier gezeigten Rollmembranen vorgesehen. In den Fig. 22 bis 44 sind die Dichtungen (Dichtungen 23, 23') nicht explizit gezeigt, sind aber trotzdem erforderlich, um die Druckräume zu definieren, wie oben beschrieben.

Mit den Druckräumen 16, 16' verbunden sind Saugventile 26, 26' sowie Druckventile 27, 27'. Die Saugventile 26, 26' stehen über einen Fluideinlass 6 mit einem Vorratsbehälter 9 für das Arbeitsfluid in Verbindung. Die Druckventile 27, 27' stehen über einen Fluidauslass 7 mit einem Druckschlauch 5 in Verbindung, der zu einem Applikator 8 führt. Die Pumpeneinheit 20 bildet zusammen mit dem Vorratsbehälter 9 samt seinem Inhalt, dem Druckschlauch 5 und dem Applikator 8 ein Einwegteil E, das nach jeder Operation entsorgt wird, so dass die Gesamtanordnung den allerhöchsten Sterilitätsanforderungen genügt.

Die Ventile gemäß der zweiten Ausführungsform werden nicht explizit beschrieben, da die Prinzipien im Wesentlichen denjenigen gemäß der ersten Ausführungsform entsprechen.

Ein Klemmventil 14 ist vorgesehen, über welches (zusätzlich zur Motorsteuerung 15) ein vollständiges Abstellen des Fluidflusses durch die Bedienungsperson B geschehen kann.

Die in Figur 1 gezeigte Ausführungsform der Erfindung weist ein Druckregelventil 35 auf, das mittels einer Ventilmembran 36 einen Verbindungskanal zwischen dem Fluidauslass 7 und dem Fluideinlass 6 öffnen und schließen kann. Die Membran 36 wird über eine Stößelstange 34 und eine Feder 33 sowie einen Kraftmesser 31 von einem Stellmotor 30 betätigt. Der Kraftmesser 31 liefert ein kraftproportionales Ausgangssignal an einen Regler 32, über welchen eine Bedienungsperson B einen Maximaldruck vorgeben kann. Statt eines gesonderten Kraftmessers 31 kann auch ein Betätigungsstrom des Stellmotors 30 gemessen werden, der ebenfalls kraftproportional ist.

Durch diese Anordnung ist gewährleistet, dass der Fluiddruck am Applikator 8 exakt eingestellt werden kann. Darüber hinaus werden durch die Kolbenbetätigung auftretende Druckschwankungen durch das Regelventil 35 ausgeglichen. Ein wesentlicher Punkt hierbei liegt darin, dass das Druckregelventil 35 aufgrund seiner Konstruktion mit einer vom Fluiddruck beaufschlagten Membran kraftgesteuert arbeitet und nicht weggesteuert. Dadurch kann bei einem Ankoppeln der Pumpeneinheit 20 an die Pumpenbetätigungseinrichtung 10 auch bei Maßtoleranzen kein Druckeinstellungsfehler auftreten, da es nicht auf die geometrischen Maße (also den Weg), sondern vielmehr auf die Kraft ankommt, mit welcher das Druckregelventil 35 betätigt wird.

Auch die soeben beschriebene Anordnung kann auf die zweite Ausführungsform übertragen werden.

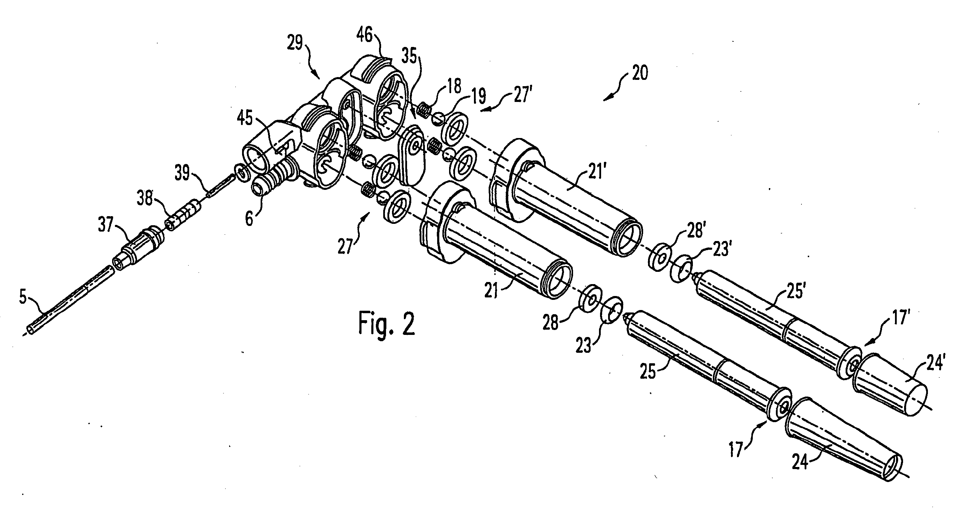

In Fig. 2 ist eine konstruktive Ausführungsform der Pumpeneinheit 20 in einer perspektivischen Explosionsdarstellung gezeigt. Bei dieser Ausführungsform umfassen die Druck- und Saugventile 26/27 Kugeln 19, die über Federn 18 auf Ventilsitze (in der Abbildung nicht sichtbar) gedrückt werden, wie dies im Prinzip bekannt ist.

Der Zylinderkopf 29 weist zwei Abschnitte zum Ankoppeln der Zylinder 21, 21' auf, wobei die Ventile zwischen den Zylindern 21, 21' und dem Zylinderkopf 29 sitzen.

Weiterhin ist aus der Darstellung nach Figur 2 ersichtlich, dass die Kolbenstangen 25, 25' an ihren distalen Enden Koppelvorsprünge 17, 17' aufweisen, über welche eine mechanische Verbindung mit den Kupplungssystemen 13, 13' bewerkstelligt wird.

Die Kolben werden bei dieser Ausführungsform der Erfindung durch proximale Enden der Kolbenstangen 25, 25' mit aufgesetzten Kappen 28 gebildet, welche gleichzeitig die Dichtungen 23, 23' fest auf den Kolbenstangen 25, 25' halten.

Der Druckschlauch 5 wird am Zylinderkopf 29 über einen Überwurfstutzen 37, ein Crimprohr 38 und ein in den Druckschlauch 5 einzusetzendes Innenrohr irreversibel befestigt, wobei nach einem Zusammenstecken (in an sich bekannter Weise) der Überwurfstutzen 37 im Zylinderkopf 29 mittels einer Schnappzunge 45 gehalten wird, die den Überwurfstutzen 37 im Zylinderkopf 29 irreversibel hält.

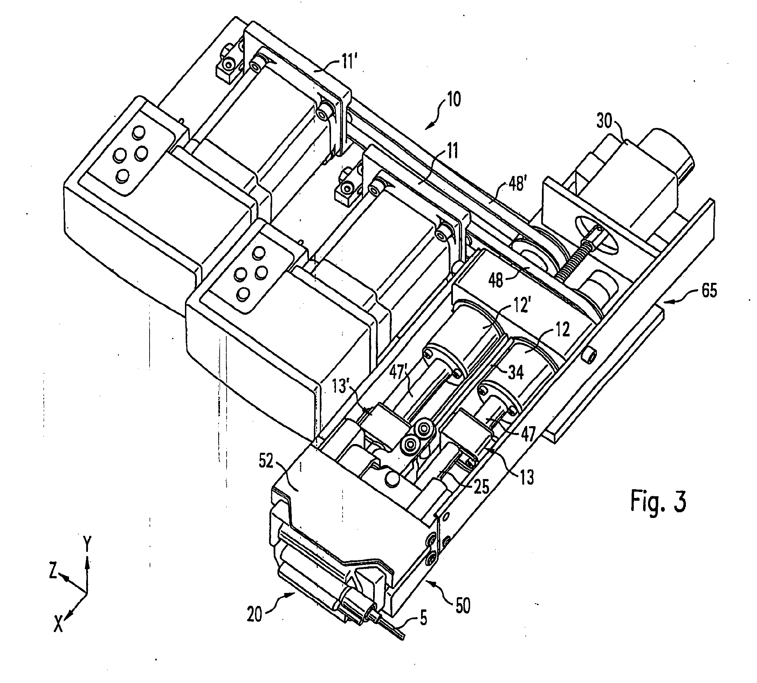

In Fig. 3 ist eine perspektivische Darstellung der Pumpenbetätigungseinrichtung 10 mit einer angekoppelten Pumpeneinheit 20 gezeigt. Aus dieser Abbildung geht hervor, dass die Pumpenbetätigungseinrichtung einen Rahmen 65 aufweist, an welchem die Motoren 11, 11' befestigt sind. Diese sind als umsteuerbare Motoren ausgebildet, welche über Zahnriemen 48, 48' und die Getriebe 12, 12' Spindeln 47, 47' antreiben, so dass die Drehbewegung der Motoren 11, 11' in Linearbewegungen umgesetzt wird. An den Spindeln 47, 47' sind die Kupplungen 13, 13' befestigt, an welche die Kolbenstangen 25, 25' ankoppelbar sind. Weiterhin ist aus dieser Abbildung die Anordnung des Stellmotors 30 mit der dazu gehörigen Stößelstange 34 ersichtlich.

Der Rahmen (Rahmen 65) ist mit den Fig. 22 bis 44 nicht explizit gezeigt, um die Komponenten der medizinischen Pumpe besser darstellen zu können. Auch in der Ausführungsform gemäß der Fig. 22 bis 44 ist jedoch ein Rahmen bzw. Gehäuse vorgesehen, das die wesentlichen Komponenten aufnimmt.

Detektionsmittel sind in dieser Ausführungsform nicht explizit abgebildet, sind jedoch auch hier vorgesehen, um die Motoren derart anzusteuern, dass ein Arbeitszustand und ein Ruhezustand gebildet werden können. Dies wird weiter unten noch näher erläutert. Explizite Ausgestaltungen der Detektionsmittel werden mit der zweiten Ausführungsform näher beschrieben.

Am Rahmen 65 ist weiterhin die Halteeinrichtung 50 angebracht, welche zum Festhalten der Pumpeneinheit 20 vorgesehen ist.

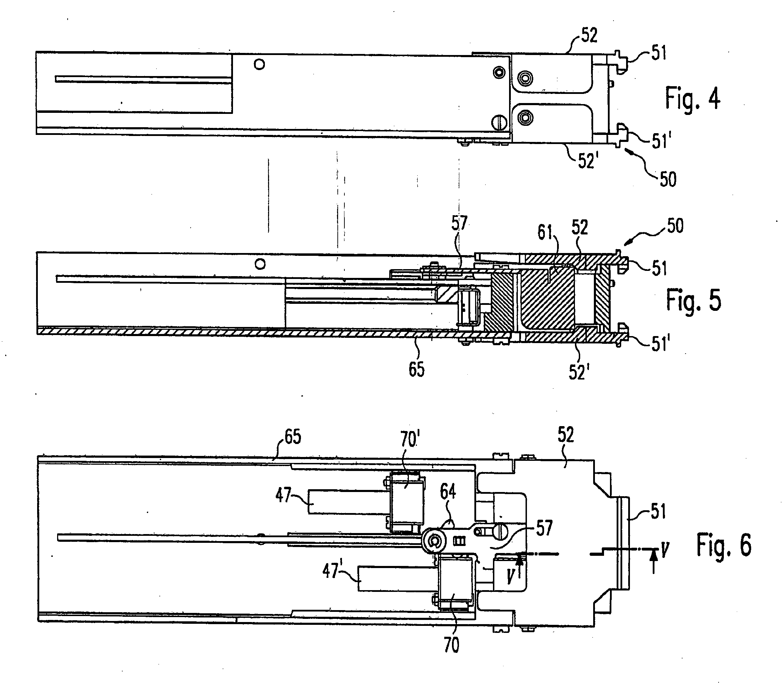

Die Halteeinrichtung 50 wird nachfolgend anhand der Fig. 4 - 12 näher erläutert.

Die Halteeinrichtung 50 umfasst Klauenhalter 52, 52' mit endseitigen Klauen 51, 51', die derart ausgebildet sind, dass sie zum Festhalten der Pumpeneinheit 20 mit den dort vorgesehenen Haltevorsprüngen 46 in Eingriff gelangen können.

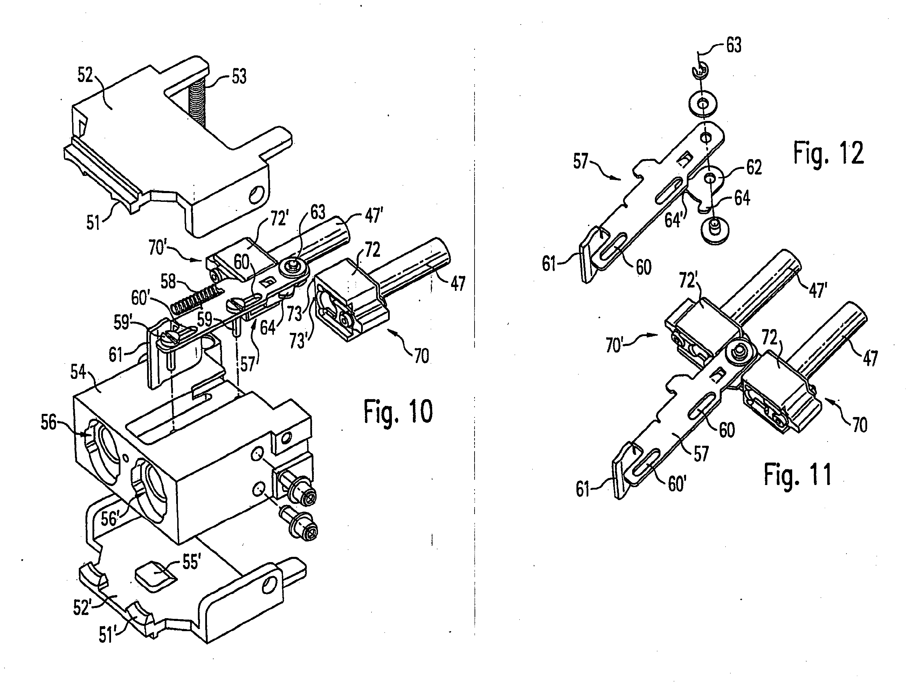

Die Klauenhalter 52, 52' sind - wie in Fig. 4 gezeichnet - über Drehachsen am Rahmen 65 gelagert und mittels Federn 53 (siehe Fig. 10) in Schließstellung (Fig. 4 - 6) vorgespannt. Zum Einsetzen einer Pumpeneinheit 20 wird diese so in die Halteeinrichtung 50 gedrückt, dass die Klauen 51, 51' mit vorderseitigen Schrägflächen über die Haltevorsprünge 46 der Pumpeneinheit 20 gleiten und aufgezwungen werden. Sobald die Pumpeneinheit 20 dann ganz angedrückt ist, schnappen die Klauenhalter 52, 52' zu und die Klauen 51, 51' halten die Pumpeneinheit 20 in dieser Position, bis sie wieder auseinander gedrückt werden.

Der Mechanismus zum Öffnen der Halteeinrichtung 50 bzw. der Klauen 51, 51' wird nachfolgend anhand der Fig. 10 - 12 erläutert.

Die Halteeinrichtung 50 umfasst einen Halteblock 54, der an seiner Vorderseite Zylinderaufnahmen 56, 56' aufweist, welche den Hinterseiten der Zylinder 21, 21' der Pumpeneinheit 20 entsprechen. Die Passform ist aus einem Vergleich der Fig. 2 und 10 leicht ersichtlich.

An der Zylinderaufnahme 56 ist ein Öffnungsschieber 57 über Befestigungsschrauben 59, 59' befestigt, wobei der Öffnungsschieber 57 Langlöcher 60, 60' aufweist, so dass er vor- und zurückverschiebbar ist. Mittels einer Feder 58 wird der Öffnungsschieber 52 nach hinten, also fort von der Pumpeneinheit 20 gedrückt.

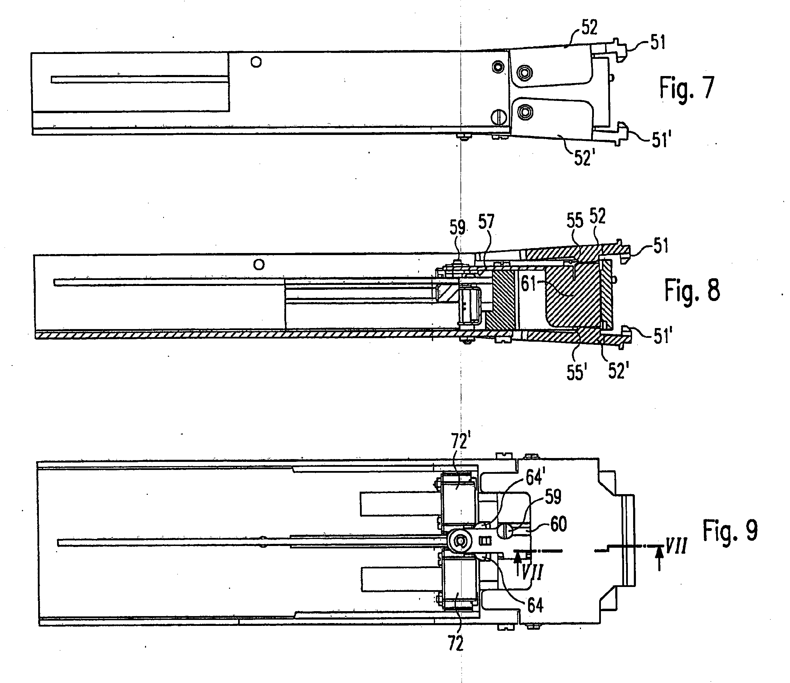

Am Öffnungsschieber 57 ist über ein Kipphebellager 63 ein Kipphebel 62 hin und her verschwenkbar angebracht, der symmetrisch angeordnete Zungen 64, 64 trägt. An seinem Vorderende, welches der Pumpeneinheit 20 zugewandt ist, trägt der Öffnungsschieber 57 eine Öffnungszunge 61. Die Öffnungszunge 61 weist nun eine Höhe auf, welche dem Abstand zwischen den Innenflächen der Klauenhalter 52, 52' entspricht. An diesen Innenflächen der Klauenhalter 52, 52' sind Öffnungsrampen 55, 55' im Verschiebeweg der Öffnungszunge 61 derart angebracht, dass die Öffnungszunge 61 beim Auftreffen auf die Öffnungsrampen 55, 55' und beim weiteren Verschieben in Richtung auf die Pumpeneinheit 50 die Klauenhalter 52, 52' auseinander drückt, so dass sie von der in den Fig. 4 - 6 gezeigten Position übergehen in die in den Fig. 7 - 9 gezeigte Position. In dieser Position (gemäß Fig. 7 - 9) kommen die Klauen 51, 51' außer Eingriff mit den Haltevorsprüngen 46 an der Pumpeneinheit 20 und geben diese somit frei.

Das Verschieben des Öffnungsschiebers 57 geschieht nun wie nachfolgend beschrieben.

Bei einer "normalen" Betätigung der Pumpeneinheit 20 werden die Spindeln 47, 47' alternierend hin- und herbewegt, so dass sie in einer Endposition einer Spindel 47 bzw. 47' die in den Fig. 6 oder 10 gezeigten Positionen einnehmen. Bei diesen Bewegungen werden Halteblöcke 72 von 72' von Kolbenhaltern 70, 70' an den Enden der Spindeln 47, 47' so an den Zungen 64, 64' vorbei bewegt, dass der Kipphebel 62 entweder in einer Richtung entgegen dem Uhrzeigersinn verkippt wird, wie dies in den Fig. 6 oder 10 gezeigt ist oder in die andere Richtung, bei welcher die Halteblöcke 72, 72' in der umgekehrt -vorgezogenen bzw. zurückgezogenen Position sind. Diese alternierenden Bewegungen der Kolbenhalter 70, 70' bzw. der Halteblöcke 72, 72' können also zur Betätigung der Pumpe ohne Verschieben des Öffnungsschiebers 57 in Richtung auf die Pumpeneinheit 20 durchgeführt werden.

Dann aber, wenn die Spindeln 47, 47' derart angetrieben werden, dass beide Kolbenhalter 70, 70' bzw. Haltblöcke 72, 72' nebeneinander laufen, kann beim Vorschieben (in Richtung auf die Pumpeneinheit 20) der Kipphebel 62 nicht ausweichen, so dass beide Halteblöcke 72, 72' mit beiden Haltezungen 64, 64' gleichzeitig in Eingriff gelangen. Durch diesen Eingriff wird nun bei einem weiteren Vorbewegen der Kolbenhalter 70, 70' der Öffnungsschieber 57 entgegen der Kraft der Feder 58 in Richtung auf die Pumpeneinheit 50 in seinen Langlöchern 60, 60' verschoben, so dass die Öffnungszunge 61 über die Öffnungsrampen 55, 55' gleitet und damit die Klauenhalter 52, 52' auseinander zwängt. Dadurch wird der Eingriff der Klauen 51, 51' gegenüber den Haltevorsprüngen 46 an der Pumpeneinheit 20 gelöst. Dieses Öffnen der Halteeinrichtung 50 geschieht also ausschließlich über die Motoren 11, 11' und deren entsprechende Steuerung durch die Motorsteuerung 15.

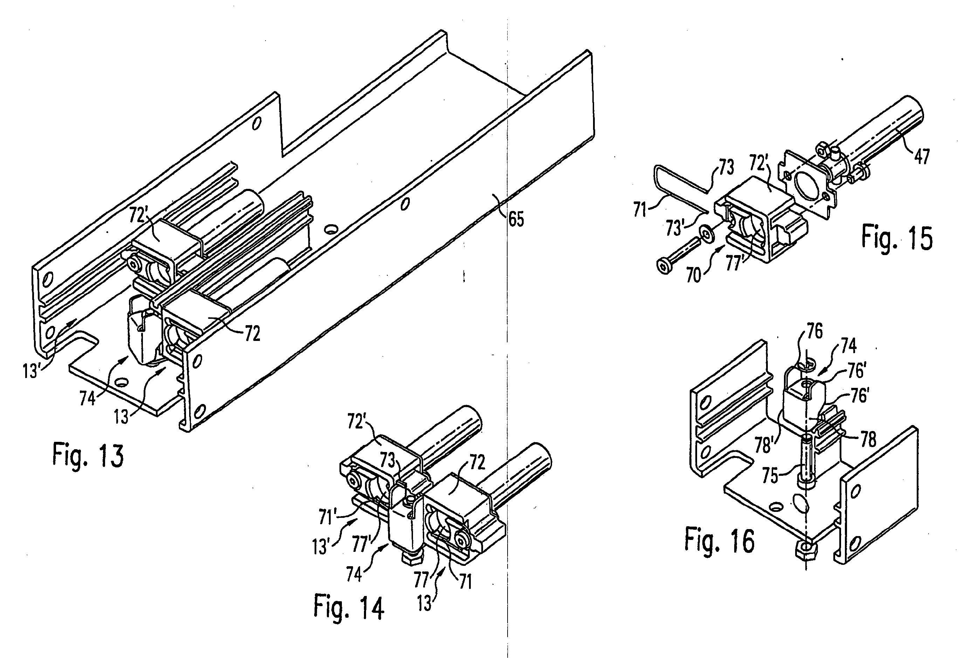

Nachfolgend wird die Wirkung bzw. Betätigung der Kupplungssysteme 13, 13' näher beschrieben, mit welcher die Kolbenstangen 25, 25' über ihre Koppelvorsprünge 17, 17' an die Kolbenhalter 70, 70' angekoppelt werden. Hierzu wird auf die Fig. 13 - 21 verwiesen.

Die Halteblöcke 72, 72' sind gemäß Fig. 15 an die Spindeln 47, 47' angeschraubt und weisen Einsetzöffnungen 77, 77' auf, in welche die Kolbenstangen 25, 25' mit ihren Koppelvorsprüngen 17, 17' eingesetzt werden können. An den Halteblöcken 72, 72' sind Federn 71, 71' so befestigt, dass Federenden 73, 73' in die Einsetzöffnungen 77, 77' ragen. Der Abstand der Federenden 73, 73' ist derart, dass die Kolbenstangen 25, 25' mit ihren Koppelvorsprüngen 17, 17' in die Einsetzöffnungen 77, 77' eingesetzt werden können und dabei die Federenden 73, 73' auseinander zwingen, bis sie hinter den Koppelvorsprüngen 17, 17' zusammenschnappen. Hierfür sind die Koppelvorsprünge 17, 17' an ihren Enden konisch ausgebildet. Nach dem Einsetzen der Koppelvorsprünge 17, 17' in die Kolbenhalter 70, 70' sind die Kolbenstangen 25, 25' schub- und zugfest mit den Kolbenhalter 70 verbunden.

Zwischen den Verschiebewegen der Kolbenhalter 70, 70' bzw. der Halteblöcke 72, 72' ist auf einem Schwenklager 75 ein Spreizhebel 74 verschwenkbar angebracht, der an seiner Ober- und Unterseite und zwar an dem der Pumpeneinheit 20 abgewandten Seite Spreizflächen 76, 76' aufweist. Am anderen, also der Pumpeneinheit 20 zugewandten Ende sind Schwenkkanten 78, 78' am Spreizhebel 74 vorgesehen.

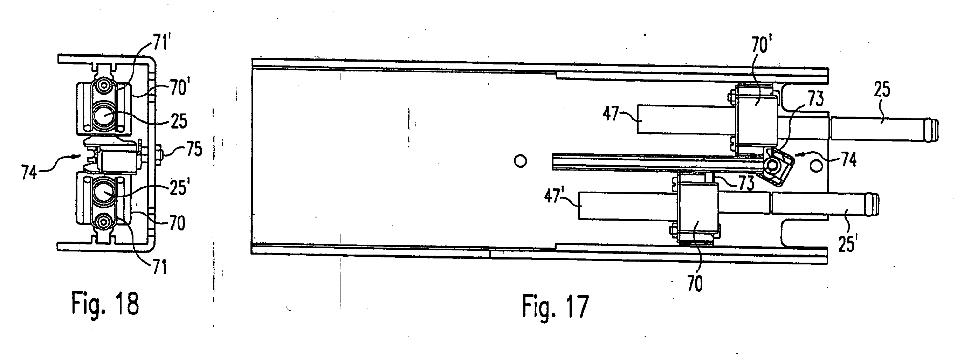

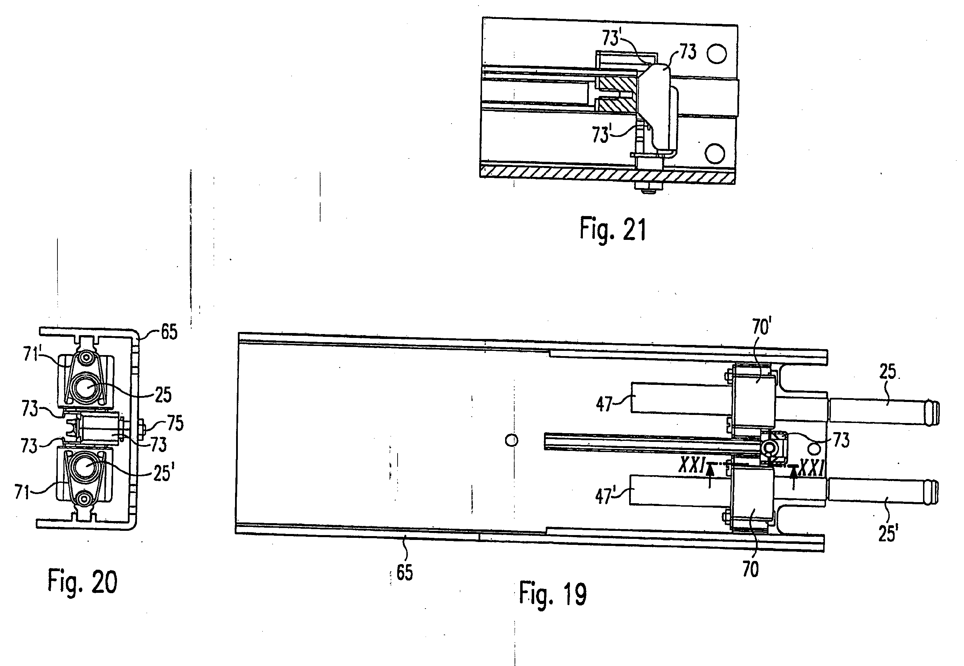

Die Anordnung und Dimensionierung des Spreizhebels 74 mit seinen Spreizflächen 76, 76' und Schwenkkanten 78, 78' ist nun derart getroffen, dass bei einer alternierenden Bewegung der Halteblöcke 72, 72' bzw. Kolbenhalter 70, 70', wie sie in den Fig. 13 und 17 gezeigt ist, der Spreizhebel 74 entweder nach der einen oder nach der anderen Seite verkippt wird, je nachdem, welcher der Kolbenhalter 70, 70' bzw. Halteblöcke 72, 72' auf seinem Weg in Richtung auf die Pumpeneinheit 20 an ihm vorbei gleitet. Durch dieses Verschwenken werden die Spreizflächen 76, 76' so verschwenkt, dass sie nicht in Eingriff mit den Federenden 73, 73' des vorbei gleitenden Kolbenhalters 70 bzw. 70' gelangen können. Dann aber, wenn die beiden Kolbenhalter 70, 70' bzw. Halteblöcke 72, 72' parallel nebeneinander in Richtung auf die Pumpeneinheit 20 verschoben werden (siehe Fig. 14 und 19 - 21) gelangen die Spreizflächen 76 bzw. 76' mit den Federenden 73, 73' in Eingriff (siehe insbesondere Fig. 20 und 21), so dass diese auf den (angeschrägten) Spreizflächen 76 bzw. 76' entlang gleiten und auseinandergezwängt werden. Durch dieses Auseinanderzwängen werden dann die zuvor an ihren Koppelvorsprüngen 17, 17' festgehaltenen Kolbenstangen 25, 25' (siehe Haltposition gemäß Fig. 18) freigegeben, wie dies in Fig. 20 gezeigt ist. Nachdem bei eben derselben gleichzeitigen und parallelen Bewegung der Kolbenhalter 70, 70' bzw. Halteblöcke 72, 72' auch die Halteeinrichtung 50 geöffnet bzw. die Klauen 51, 51' auseinandergezwängt und damit deren Eingriff mit den Haltevorsprüngen 46 der Pumpeneinheit 20 aufgehoben wird, kann die Pumpeneinheit bei dem parallelen Verschieben der Kolbenhalter 70, 70' bis zu ihrer vorderen, der Pumpeneinheit 20 zugewandten Position ohne Überwindung von Kraft durch den Benutzer entfernt werden.

Die Motorsteuerung 15 ist weiterhin derart ausgebildet, dass nach Abnehmen einer Pumpeneinheit 20 von der Pumpenbetätigungseinrichtung 10 die beiden Spindeln 47, 47' die Kolbenhalter 70, 70' zurückziehen. Setzt also der Benutzer eine Pumpeneinheit 20 in die Pumpenbetätigungseinrichtung 10 ein, so muss er lediglich die zum Öffnen der Halteeinrichtung 50 notwendige Kraft überwinden. Die Kolbenstangen 25, 25' ragen dann mit ihren Koppelvorsprüngen 17, 17' durch die Zylinderaufnahmen 56, 56' in die Pumpenbetätigungseinrichtung 10 hinein. Der Benutzer kann nun- die Motorsteuerung 15 derart ansteuern, dass diese in einem "Kopplungsmodus" die Kolbenhalter 70, 70' in Richtung auf die Pumpeneinheit 20 zu bewegt, bis die Koppelvorsprünge 17 bzw. 17' die Federenden 73, 73' auseinander drücken und einschnappen. Dieser Einschnappvorgang wird getrennt für die beiden Koppelvorsprünge 17 bzw. 17' nacheinander vorgenommen, so dass der Spreizhebel 74 die Federn 71, 71' nicht öffnet.

In diesem Falle wird also das Einsetzen der Pumpeneinheit 20 in die Halteeinrichtung 50 detektiert. Ein aufgrund der Detektion generiertes Detektionssignal D, D' (Pumpeneinheit befindet sich in der Halteeinrichtung) veranlasst die Steuerung, die Motoren derart anzusteuern, dass der Kopplungsmodus selbsttätig durchführbar ist, d. h., die Ankopplung der Kolben in der Pumpenbetätigungseinrichtung und damit die Ausbildung eines Arbeitszustandes wird selbsttätig durchgeführt. Die Detektionsmittel können z. B. als eine Lichtschranke ausgebildet sein. Einzelheiten hierzu werden mit der zweiten Ausführungsform näher beschrieben.

Nachfolgend wird die zweite Ausführungsform mit den Fig. 22 bis 44 beschrieben. Das Einsetzen und auch das Entfernen der Pumpeneinheit bzw. Pumpeinheit in die bzw. aus der Pumpenbetätigungseinrichtung erfolgt im Wesentlichen nach den Prinzipien, wie sie bereits mit der Ausführungsform gemäß der Fig. 2 bis 21 beschrieben wurden. Unterschiedliche Ausgestaltungen einzelner Bauteile der zweiten Ausführungsform und eine ggf. unterschiedliche Wirkungsweise werden jedoch im Einzelnen dargestellt. So sind z. B. bei der zweiten Ausführungsform Detektionsmittel bzw. Sensoreinrichtungen gezeigt, die eine weitere Erleichterung der Handhabung der medizinischen Pumpe für den Bediener ermöglichen. Es sei jedoch erwähnt, dass die Detektionsmittel ebenfalls mit der medizinischen Pumpe gemäß der ersten Ausführungsform vorgesehen sind.

Die Anordnung der Ventile und deren Wirkungsweise in Verbindung mit Vorratsbehälter, Applikator und Schlauchverbindungen (z. B. Druckschlauch) werden in der zweiten Ausführungsform nicht mehr explizit beschrieben. Hier sei auf die erste Ausführungsform verwiesen. Gleiches gilt für die Regulierung des Fluidflusses und des Druckes. Vorratsbehälter und Applikator werden mit der zweiten Ausführungsform nicht explizit gezeigt, hier sei z. B. auf Fig. 1 verwiesen.

Fig. 22 zeigt eine perspektivische Darstellung der Pumpenbetätigungseinrichtung 100 mit noch nicht angekoppelter Pumpeneinheit 200. Anhand dieser Abbildung sollen weitere Details von Pumpenbetätigungseinrichtung 100 und Pumpeneinheit 200 erläutert werden.

Die Pumpenbetätigungseinrichtung 100 weist Motoren 110, 110' auf, welche über Zahnriemen 480, 480' und Getriebe 120, 120' Spindeln (hier nicht sichtbar) antreiben, so dass die Drehbewegung der Motoren 110, 110' in Linearbewegungen umgesetzt und so eine Kolbenbewegung erreicht wird. Motoren und Getriebe bilden Antriebseinrichtungen aus. Gesteuert werden die Motoren über eine Steuerungseinrichtung bzw. Steuerung 150. Die Spindeln sind in Spindelaufnahmen 471, 471' eingesetzt, wobei diese über einen Lagerbock 652 gelagert sind. Der Lagerbock 652 ist auf einer Grundplatte 651 angeordnet, die ebenfalls die Motoren 110, 110' aufnimmt. An der Grundplatte ist zudem ein Rahmen (in den Fig. 22 bis 44 nicht gezeigt) angeordnet, der die Spindelaufnahmen aufnimmt. Die Spindeln sind an Kupplungseinrichtungen 130, 130' angebracht, wobei das Kupplungssystem Halteblöcke 720, 720' eines Kolbenhalters 700 aufweist. Durch die Bewegung der Spindeln über die Motoren lassen sich die Kolbenhalter mit den Halteblöcken mitbewegen.

Die Pumpenbetätigungseinrichtung 100 weist ferner eine Halteeinrichtung 500 auf, welche zum Festhalten der Pumpeneinheit 200 vorgesehen ist. Die Halteeinrichtung 500 besteht im Wesentlichen aus einer Aufnahmeeinrichtung 510 mit einem Aufnahmerahmen 520. Die Halteeinrichtung 500 umfasst auch hier Zylinderaufnahmen 560, 560', wie mit der ersten Ausführungsform beschrieben. In dem Aufnahmerahmen 520 sind quer, innerhalb des Rahmens verlaufende, jeweils an einer Oberseite und einer Unterseite des Rahmens angeordnete Achsbolzen 502, 502' vorgesehen, die mit ihren Enden über den Rahmen an seinen Seitenflächen hervorstehen. Die Achsbolzen sind in Nuten des Rahmens eingesetzt und können insofern im Rahmen im Wesentlichen versenkt werden. Die sich jeweils an einer Seitenfläche des Rahmens 520 gegenüberliegenden Enden der Achsbolzen 502, 502' stehen aus dem Rahmen über Ausnehmungen hervor und sind mit Zugfedern 503, 503' miteinander verbunden. Ferner ist die Halteeinrichtung 500 mit Ausrückhebeln 501, 501' ausgebildet, welche über an der Aufnahmeeinrichtung gelagerte Zylinderstifte 504, 504' als Drehachsen auf- und zuklappbar sind und die Aufnahmeeinrichtung 510 jeweils an ihrer Oberseite und Unterseite überdecken (die Zylinderstifte sind beispielsweise über Gleitlager gelagert). Damit schließen die Ausrückhebel die Aufnahmeeinrichtung im Wesentlichen maulförmig ein. An Auslegern der Ausrückhebel sind die Achsbolzen 502, 502' mit ihren jeweiligen Enden gelagert und werden über die Ausrückhebel aufeinander zu oder voneinander wegbewegt (eben maulförmig), also mitgenommen, das heißt, aus den Nuten gezogen oder in diese wieder eingeschoben).

Wie der Abbildung weiter zu entnehmen ist, ist an der Oberseite der Halteeinrichtung 500 eine Lichtschranke, hier eine Gabellichtschranke 901 angeordnet, die im Wesentlichen in das Innere der Halteinrichtung 500 ragt. Die Lichtschranke 901 ist mit der Steuerung 150, die auch die Motorsteuerung übernimmt, über ein Steckerelement 902 verbindbar. Ferner ist, ebenfalls mit der Steuerung 150 verbindbar, ein Mikroschalter 801 an einer Seitenwand der Aufnahmeeinrichtung 510 vorgesehen, wobei dessen Betätigungsstift 803 ebenfalls in das Innere der Halteeinrichtung 500 ragt. Der Betätigungsstift 803 ist über eine Druckfeder 802 betätigbar und stellt der Steuerung 150 aufgrund der Betätigung oder Nicht-Betätigung ein entsprechendes Signal bereit. Beide Detektionsmittel (Lichtschranke und Mikroschalter) übermitteln ein generiertes Signal (erstes und zweites Detektionssignal D, D') an die Steuerung 150, so dass diese aufgrund der Signale die Motoren ansteuert. Die Motoren ermöglichen dann schließlich die Verfahrbewegung der Kolbenhalter bzw. der Halteblöcke und die Ausrichtung der Halteeinrichtung, um ein Verbinden von Pumpeneinheit 200 und Pumpenbetätigungseinrichtung 100 und auch ein Loslösen zu ermöglichen. Einzelheiten hierzu werden nachfolgend noch detaillierter beschrieben.

Die Pumpeneinheit 200 weist in dieser Ausführungsform einen Zylinderblock 211 auf, in welchen Zylinder 210, 210' angeordnet sind und der in diesem Falle integral mit einem Ventildeckel 271 ausgebildet ist (der Ventildeckel umschließt die mit Fig. 1 beschriebenen Saug- und Druckventile). Kolben 250, 250' (hier nicht sichtbar) sind jeweils mit einem Faltenbalg 240, 240' verbunden (wobei jeder Faltenbalg auch mit einem der Zylinder verbunden ist) und sind so außerhalb der Zylinder mittels des Faltenbalgs abgedeckt. Die jeweiligen Enden der Kolben weisen Koppelvorsprünge 170, 170' auf, über die die Kolben an den Kupplungseinrichtungen 130, 130' der Pumpenbetätig-ungseinrichtung 100 aufnehmbar sind. An dem Ventildeckel 271 ist jeweils an seiner Unter- als auch an seiner Oberseite ein quer (quer zur Flucht der Zylinder) über den Ventildeckel verlaufender (erster) Haltesteg 273, 273' angeordnet (ein zweiter, parallel dazu angeordneter Steg ist eine konstruktive Ausgestaltung des Ventildeckels). Die Haltestege 273, 273' (die in Bezug auf die jeweiligen zweiten Stege näher in Richtung der Kolben oder Zylinder angeordnet sind) sind derart ausgelegt, dass die Achsbolzen 502, 502' hinter diesen (und damit zwischen den beiden Stegen) zum Liegen kommen, wenn die Pumpeneinheit 200 in die Haltevorrichtung 500 aufgenommen ist. An dem Haltesteg 273 ist ein weiterer Steg auf der Oberseite des Ventildeckels 271, senkrecht zu dem Haltesteg und senkrecht zu der Ventildeckeloberseite angeordnet. Dieser ist als eine Unterbrechereinrichtung 272 zum Unterbrechen, z. B. Reflektieren eines Lichtstrahles der Gabellichtschranke 901 vorgesehen, sobald die Pumpeneinheit 200 in die Pumpenbetätigungseinrichtung 100 eingesetzt ist. Die genaue Wirkungsweise wird weiter unten näher ausgeführt.

Fig. 23 zeigt die Unterbrechereinrichtung 272 bzw. Unterbrecherfahne in vergrößerter Darstellung.

Wie bereits oben beschrieben, sind Detektionsmittel vorgesehen, die einen Einsetzzustand (Pumpeneinheit in Pumpenbetätigungseinrichtung eingesetzt oder nicht) erfassen, woraufhin die Steuerung die Motoren derart ansteuert, dass diese die Kolbenhalter über die Spindeln insbesondere zum Befestigen der Pumpeneinheit in der Halteeinrichtung und zum Ankoppeln der Kolben an das Kupplungssystem entsprechend verfahren. Allerdings werden auch das Entkoppeln und Lösen selbsttätig durch das System durchgeführt. Dabei durchläuft das System (d. h. die Kolbenhalter) entsprechende Positionen, die kurz beschrieben werden sollen. Wenn nachfolgend von einem "Verfahren der Halteblöcke" die Rede ist, so bedeutet dies, dass die Motoren die Spindeln verfahren und diese dabei die Kupplungseinrichtungen, d. h. die Kolbenhalter und damit die Halteblöcke mitnehmen. Gleichzeitig oder zumindest zeitlich nah wird in der jeweiligen Position auch die Halteeinrichtung entsprechend ausgerichtet, um das Befestigen oder Lösen der Pumpeneinrichtung zu ermöglichen. Die entsprechenden Positionen definieren also jeweils die Position der Halteblöcke und damit der Kupplungseinrichtungen, als auch die Ausrichtung der Halteeinrichtung. Damit nehmen Halteeinrichtung und Kupplungseinrichtungen die jeweilige Position ein.

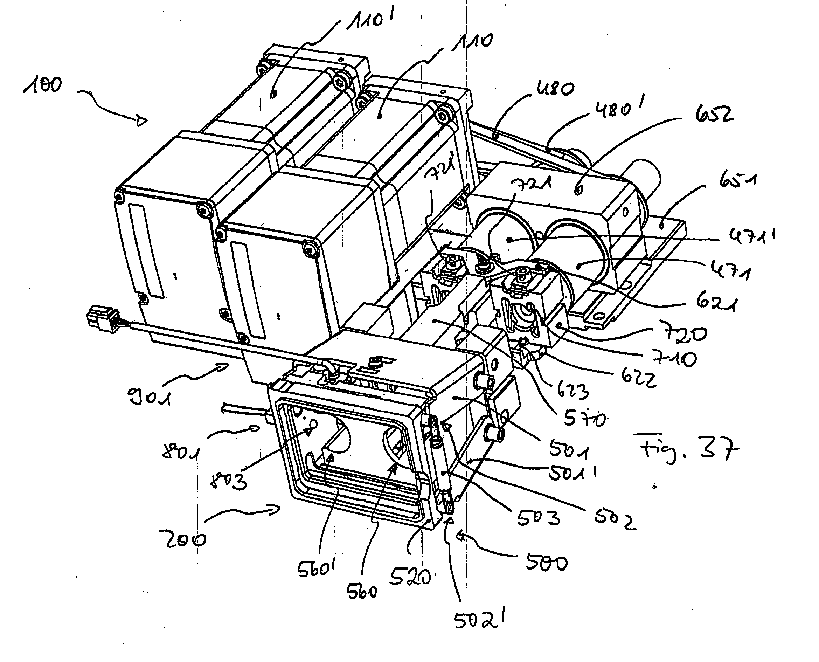

Zur Inbetriebnahme der Pumpenbetätigungseinrichtung 100 (z. B. Einschalten der Pumpe) muss diese eingeschaltet werden. Dies ist z. B. mittels eines Schalters oder eines entsprechenden Bereichs auf einem Touchscreen oder dergleichen Eingabeelement durchführbar. Nach der Aktivierung müssen die Schrittmotoren hinsichtlich ihrer Positionierung abgeglichen werden. Insofern wird eine Referenzfahrt durchgeführt und die Halteblöcke werden in eine Referenzposition verfahren. Dies bedeutet, dass die Halteblöcke derart nach hinten verfahren werden, dass sie im Wesentlichen an den Spindelaufnahmen anliegen (Fig. 37).

Anschließend werden die Halteblöcke in eine Einsetzposition verfahren. In dieser Position, die von der Halteeinrichtung aus in Richtung Spindelaufnahme gesehen vor der Referenzposition liegt, werden die Achsbolzen im Aufnahmerahmen bereits leicht aus den Nuten an der Ober- und Unterseite des Rahmens gezogen (weil die Ausrückhebel leicht schließen und durch die Zugfeder zusammengezogen werden). Die Achsbolzen werden also in den freien Raum innerhalb des Rahmens gezogen (z. B. Fig. 22).

Nun kann eine Bedienperson die Pumpeneinheit einsetzen. Der Zylinderblock ist derart ausgebildet, dass er die Achsbolzen mit entsprechend zum "Einfädeln" abgeschrägten Seitenflächen wieder leicht auseinanderdrückt, diese also kurzfristig wieder in die Nuten zurückschiebt, so dass die Haltestege 273, 273' über die Nuten in den Aufnahmerahmen geschoben werden können. Sobald sich die Haltestege in dem Aufnahmerahmen befinden und die Seitenflächen des Zylinderblocks die Achsbolzen wieder freigeben (eben weil die an den Haltestegen angeordneten Seitenflächen mit den Stegen abschließen), werden die Achsbolzen 502, 502' schließlich hinter den Haltestegen 273, 273' aufgrund der Kraft der Zugfeder wieder aus den Nuten geholt und hinter den Haltestegen (zwischen den beiden jeweiligen Stegen) positioniert. Die Pumpeneinheit befindet sich nun in einem vorverriegelten Zustand und kann während des Ankoppelns der Kolben nicht mehr aus der Halteeinrichtung geschoben werden. Das heißt, die Achsbolzen sind derart hinter der den Haltestegen angeordnet, dass durch das Zusammenwirken von Haltesteg und Achsbolzen eine Entnahme der Pumpeneinheit 200 verhindert wird.

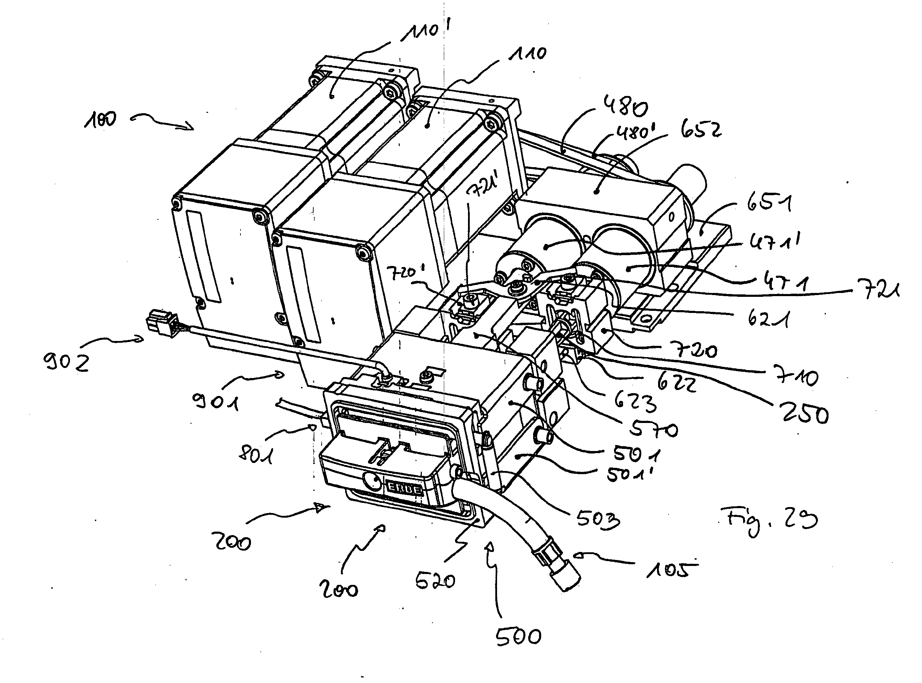

Das Einschieben der Pumpeneinheit wird durch die oben bereits beschriebenen Detektionsmittel delektiert, so dass die Halteblöcke aufgrund des somit generierten bzw. von den Detektionsmitteln bereitgestellten ersten Detektionssignals in eine Befestigungs- und Ankoppelposition verfahren werden. Diese befindet sich wiederum vor der Einsetzposition, wobei die Halteblöcke im Wesentlichen an der Halteeinrichtung anliegen. In dieser Position werden die Kolben der Pumpeneinheit (wie unten näher beschrieben) an das Kupplungssystem bzw. die Halteblöcke angekoppelt. Im Wesentlichen gleichzeitig (ggf. zeitlich auch geringfügig versetzt) werden die Ausrückhebel durch die Verfahrbewegung nach vorne vollständig geschlossen, so dass die Pumpeneinheit in der Halteeinrichtung sicher befestigt ist. Nun ist die medizinische Pumpe für den Betrieb bereit (Pumpenbetrieb z. B. Fig. 29).

Die Halteblöcke werden nun in eine Stand-by-Position-verfahren, die zwischen der Einsetzposition und der Befestigungs- und Ankoppelposition liegt. Die Befestigungs- bzw. Ankoppekoutine ist beendet bzw. endet, wenn die Halteblöcke mit den Kolben in der für den Pumpenbetrieb geeigneten und durch die Steuerung (Softwaresteuerung) in diese vordefinierte Stand-by-Position verfahren worden sind. Aus dieser Position heraus kann die Bedienperson durch Betätigung eines Hand-, Fußschalters oder dergleichen Schalter oder Taster den eigentlichen Pumpenbetrieb auslösen.

Durch Loslassen des Fuß- oder Handschalters (auch Fingerschalter oder dergleichen Schaltelement) wird die Beendigung des Pumpenbetriebs angezeigt. Der Pumpenbetrieb wird also solange ausgeführt, solange der Fuß- oder Handschalter betätigt ist. Grundsätzlich ließe sich auch durch ein erneutes Betätigen des jeweiligen Schalters die gewünschte Beendigung des Pumpenbetriebs anzeigen. Nun kann der Bediener entscheiden, ob er mit dem Pumpenbetrieb weiter fortfahren will (erneute Betätigung des Hand- oder Fußschalters) oder ob die Pumpeneinheit aus der Pumpenbetätigungseinrichtung entfernt werden soll.

Da die Pumpeneinheit und eventuell auch der Zuleitungsschlauch vom Vorratsbehälter des Fluids (Trennmediums) zur Pumpeneinheit nach dem Einsetzen (aber vor Inbetriebnahme) derselben in die Halteeinrichtung noch nicht mit dem Fluid gefüllt sind und so ein sofortiger Einsatz der medizinischen Pumpe nicht gewährleistet ist, könnte nach dem Befestigungs- und Ankoppelvorgang ein automatischer Füll- bzw. Ansaugvorgang folgen. Dies hätte den Vorteil, dass die Zeit, bis das Fluid beim erstmaligen Aktivieren tatsächlich am Applikator verfügbar ist, deutlich verkürzt werden könnte. Voraussetzungen für die Effizienz der Vorfüllroutine sind zumindest folgende: Der Zuleitungsschlauch muss-korrekt an der Pumpeneinheit und an dem Vorratsbehälter angeschlossen und es muss genügend Trennmedium in dem Vorratsbehälter vorhanden sein.

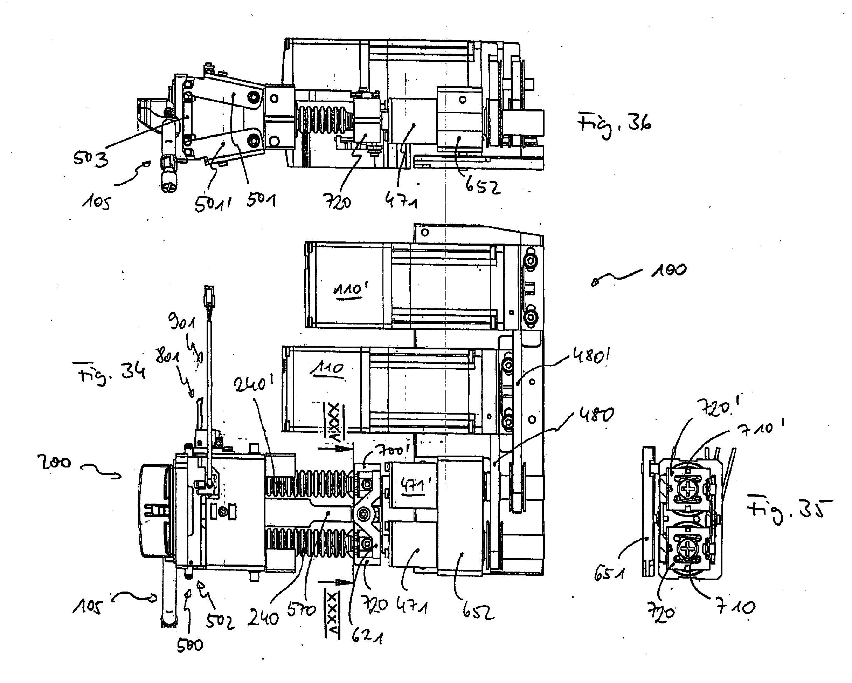

Soll ein Entfernen der Pumpeneinheit durchgeführt werden, muss der Steuerung das gewünschte Entfernen angezeigt werden. Dies geschieht z. B. durch das Betätigen einer entsprechenden Taste, eines Schalters (z. B. Eject-Taste) oder dergleichen Eingabeelement. In der Praxis kann dies auch durch das Berühren eines entsprechend dafür vorgesehenen Bereichs auf einem Touchscreen durchgeführt werden. Die Motoren werden von der Steuerung entsprechend angesteuert, so dass die Halteblöcke, durch das Anzeigen, dass die Pumpeneinheit entfernt werden soll, wieder ganz nach hinten gefahren werden, bis sie im Wesentlichen an den Spindelaufnahmen anliegen. In dieser Position, einer Löse- und Entkoppelposition (die von der Positionierung her der Referenzposition entspricht), werden die Kolben wieder aus den Halteblöcken ausgeworfen, also entkoppelt. Unmittelbar nach dem Entkoppeln sind die Ausrückhebel wieder vollständig geöffnet, so dass die Pumpenbetätigungseinrichtung durch die Bedienperson wieder entnommen werden kann (z. B. Fig. 34 oder 36).

Auch das Entnehmen der Pumpeneinheit wird wieder detektiert. Die Halteblöcke werden aufgrund der detektierten Entnahme in die Einsetzposition verfahren. In dieser Position kann erneut eine Pumpeneinheit eingesetzt werden. Um ein erneutes, ungewolltes Befestigen und Ankoppeln der bereits benutzten Pumpeneinheit zu vermeiden, wird dieser Vorgang (also das Verfahren in die Einsetzposition) vorzugsweise mit einer Zeitverzögerung ausgeführt. Die Zeit wird hierbei so bemessen, dass der Benutzer die Pumpeneinheit problemlos in der Löse- und Entkoppelposition entfernen kann, aber auch das Einsetzen einer neuen Pumpeneinheit ohne merkliche Verzögerung erfolgen kann..Das Verfahren in die Einsetzposition entspricht der Ausbildung des Ruhezustandes.

Das Einsetzen der Pumpeneinheit 200 in die Pumpenbetätigungseinrichtung 100, das Befestigen der Pumpeneinheit in der Halteeinrichtung 500 und das Ankoppeln der Kolben 250, 250' an das Kupplungssystem 130, 130' wird nachfolgend anhand der Fig. 22 bis 28 beschrieben.

Das Pumpensystem wird in Betrieb genommen (eingeschaltet). Nacheinander werden, von der Steuerung 150 gesteuert, die Referenz- und die Einsetzposition angefahren (Halteblöcke werden entsprechend verfahren). Die Einsetzposition ist in den Fig. 22 bis 28 dargestellt. Die Einsetzposition ermöglicht die Vorverrieglung der später einzusetzenden Pumpeneinheit 200, wie oben beschrieben.

Eine Bedienperson schiebt nun die Pumpeneinheit 200 über die Zylinderaufnahmen 560, 560' in die Halteeinrichtung 500. Die Ausrückhebel 501, 501' sind in dieser Position noch geöffnet.

Durch das ordnungsgemäße Einsetzen der Pumpe durch den Bediener wird ein Lichtstrahl der Lichtschranke 901 unterbrochen (und in diesem Falle reflektiert), weil die Unterbrechereinrichtung 272 (oder Unterbrecherfahne) zwischen die Gabel der Lichtschranke eingeschoben wird. Die Lichtschranke ist derart ausgestaltet, dass sie auf das Unterbrechen des Lichtstrahls anspricht. Üblicherweise ist die Pumpeneinheit als Sterilprodukt definiert und z. B. als Kunststoffspritzgießteil konstruiert. Das Material der Gehäuseteile der Pumpeneinheit kann z. B. aus einem speziellen, durch Gammastrahlen sterilisierbaren Polycarbonat gefertigt sein. Dieses Material ist transluzent und vermag den Lichtstrahl der Lichtschranke nicht zu unterbrechen. Deshalb ist die Unterbrechereinheit 272 (als Bestandteil des Kunststoffspritzgießteils) in dieser Ausführungsform so ausgestaltet, dass der Teil, der in die Lichtschranke eintaucht eine Reflexion des von einem Sender der Lichtschranke ausgesendeten Lichts bewirkt und die Lichtschranke, hier z. B. die Gabellichtschranke 901 "auslöst". Die Unterbrechereinrichtung weist z. B. eine entsprechende Schräge auf, an der der Lichtstrahl reflektiert wird, ggf. sogar totalreflektiert wird. Das heißt die Reflexion kann auch eine Totalreflexion sein. In dieser Ausführungsform würde der Lichtstrahl auf die Unterbrechereinrichtung auftreffen, diese durchdringen und an der Schräge reflektiert werden. Das heißt, es wird ein Signal, das erste Detektionssignal D, aufgrund der Unterbrechung des Lichtstrahls generiert bzw. von der Lichtschranke bereitgestellt, wobei das Signal dann an die Steuerung 150 übermittelt wird. Diese veranlasst die Motoren, die Kolbenaufnahmen 700, 700' über die Spindeln 470, 470' wieder in Bewegung zu setzen und die Befestigungs- und Ankoppelposition anzufahren.

Gleichzeitig mit dem Einsetzen der Pumpeneinheit 200 in die Halteeinrichtung 500 wird der Mikroschalter 801 betätigt. Die Betätigung erfolgt über eine Seitenwand des Zylinderblocks 211, wobei die Seitenwand durch das Einsetzen der Pumpeneinheit letztendlich den Betätigungsstift 803 des Mikroschalters 801 drückt, also betätigt. Der Zylinderblock weist - wie bereits oben dargelegt - die abgeschrägten Seitenflächen auf, die an einem in Richtung der Kolben und der Spindelaufnahmen weisenden Ende angefast sind. Damit lassen sich die Seitenwände des Zylinderblocks, d. h. insbesondere die eine Seitenwand leicht über den Betätigungsstift des Mikroschalters zu dessen Betätigung führen. Durch Betätigung des Mikroschalters wird der Motorsteuerung 150 angezeigt, dass eine Pumpeneinheit 200 eingesetzt ist. Die genauere Bedeutung des Mikroschalters wird weiter unten noch näher erläutert.

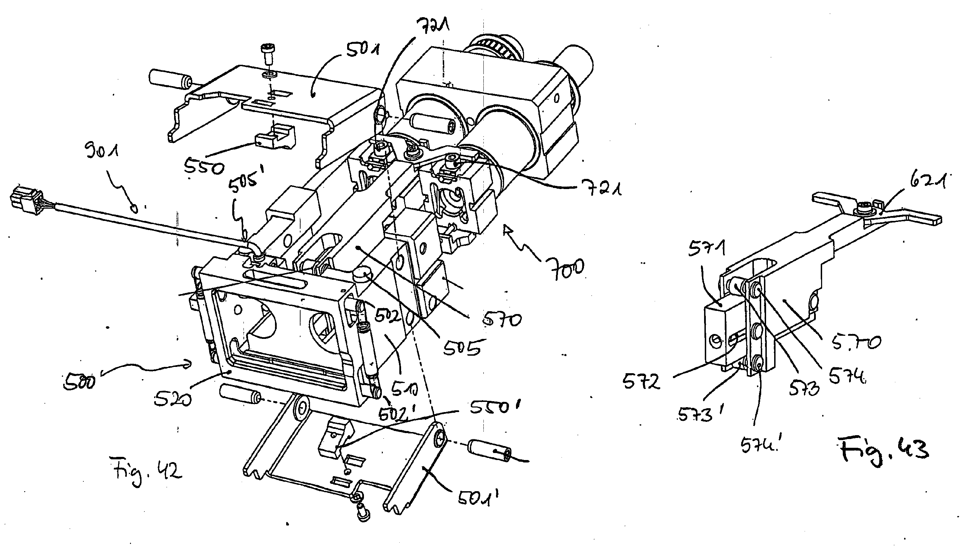

Nachfolgend wird die Betätigung der Ausrückhebel 501, 501' durch das Verfahren der Halteblöcke 720, 720' beschrieben, d. h. deren maulförmiges auf- und zuklappen (hierzu sei insbesondere auf die Fig. 42 und 43 verwiesen). Der Antrieb der Kolbenaufnahmen erfolgt - wie oben bereits erläutert - aufgrund des durch die unterbrochene Lichtschranke generierten, an die Steuerung übermittelten Signals.

An der Halteeinrichtung 500 ist ein Öffnungsschieber 570 über eine Führung 571 für den Öffnungsschieber befestigt, wobei die Führung 571 Langlöcher aufweist, so dass der Öffnungsschieber in der Führung vor- und zurückverschiebbar ist. Am Öffnungsschieber 570 ist eine Wippe 621 angebracht, die ferner über beide Halteblöcke 720, 720' gelagert ist. Dabei sind Stutzeneinrichtungen (hier Schraubenelemente) 721, 721' an einer Oberseite der Halteblöcke 720, 720' angeordnet, die die Wippe 621 und damit den Öffnungsschieber 570 u. a. dann in die jeweilige Richtung mitnehmen, wenn sich die beiden Wippenarme im Eingriff mit den Stutzeneinrichtungen befanden und sich die Kolbenhalter nicht gleichmäßig asynchron, also nicht gleichmäßig mit im Wesentlichen der gleichen Geschwindigkeit gegeneinander bewegen. Bewegen sich also z. B. beide Kolbenaufnahmen synchron bzw. im Wesentlichen parallel in dieselbe Richtung (oder zumindest so, dass kein gleichmäßig alternierender Pumpenbetrieb möglich wäre) in Richtung der Halteeinrichtung 500, also in die Befestigungs- und Ankoppelposition, so wird der Öffnungsschieber 570 ebenfalls, in der Führung 571 geführt, in Richtung der Halteeinrichtung 500 bewegt. Dies gilt natürlich auch für die umgekehrte Richtung. Im Pumpenbetrieb bei im Wesentlichen asynchroner Bewegung der Kolbenhalter wird die Wippe und damit der Öffnungsschieber nicht verschoben, da der Öffnungsschieber in der Position belassen werden soll, die den Pumpenbetrieb ermöglicht.

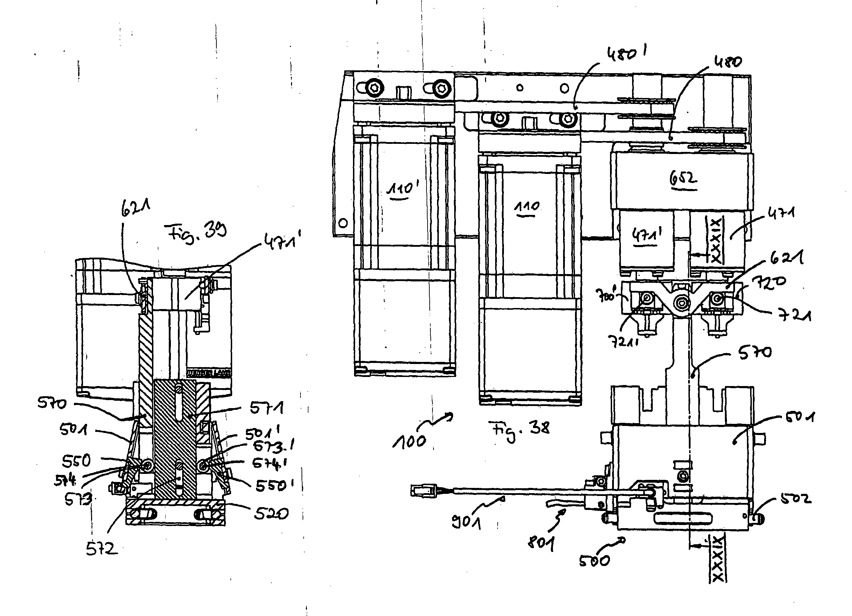

In Fig. 43 ist der Öffnungsschieber 570 im Detail dargestellt. Der Öffnungsschieber wird in der Führung über Langlöcher 572 geführt. Weiterhin sind an dem Schieber ein oberes und ein unteres, über Achsbolzen 574, 574' gelagerte Gleitlager 573, 573' angeordnet. Die Gleitlager 573, 573' des Öffnungsschiebers gleiten entlang von Innenflächen der Ausrückhebel, wobei in deren Verschiebeweg an den Innenflächen angeordnete Öffnungsrampen 550, 550' vorgesehen sind. Durch die Verfahrbewegung des Öffnungsschiebers 570 entlang der Führung 571 über die Langlöcher 572 bewegen die Gleitlager beim Auftreffen auf die Öffnungsrampen und beim weiteren Verschieben des Öffnungsschiebers 570 die Ausrückhebel 501, 501' auseinander oder wieder zusammen, so dass ein vollständiges Öffnen und Schließen der Ausrückhebel 501, 501' durchführbar ist. Ein Öffnen der Ausrückhebel wird erreicht durch das Zurückziehen der Kolbenhalter und damit der Wippe mit dem Öffnungsschieber in Richtung Referenzposition. Umgekehrt wird ein Schließen durch die entgegengesetzte Bewegung erreicht. Beim Öffnen der Ausrückhebel 501, 501' werden die Zugfedern 503, 503' gespannt und halten die Ausrückhebel zusammen. Wie in Fig. 39 gezeigt ist, befinden sich die Gleitlager auf jeweils einer maximal höchsten Ebene der Öffnungsrampen, wenn der Öffnungsschieber vollständig zurückgezogen und die Ausrückhebel maximal geöffnet sind. Die Ausrückhebel, 501, 501' werden in geschlossenem Zustand auf an der Aufnahmeeinrichtung angeordneten elastisch verformbaren Elementen, vorzugsweise Gummielementen 505, 505' gelagert, um deren Klappern zu verhindern.

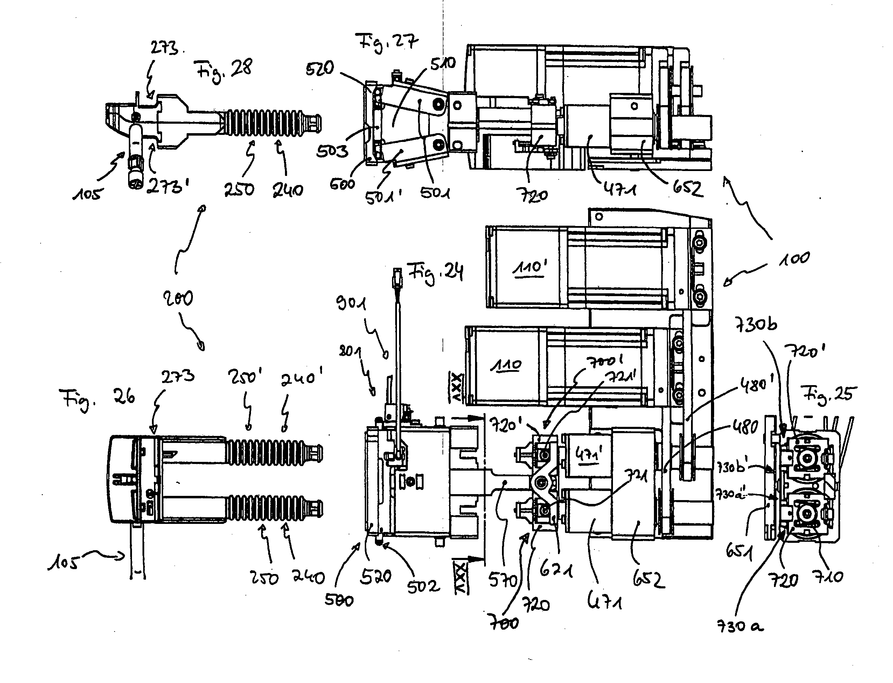

Die Fig. 24 und 26 bis 28 zeigen die perspektivische Ansicht gemäß Fig. 22 in Draufsichten bzw. Seitenansichten. Aus Fig. 24 ist insbesondere zu entnehmen, dass sich die Kolbenaufnahmen 700, 700' bzw. die Halteblöcke 720, 720' in einer zurückgezogenen Position (Einsetzposition) befinden. Damit befindet sich auch der Öffnungsschieber 570 mit der Wippe 621 in der zurückgezogenen Position. Die Ausrückhebel 501, 501' sind daher noch geöffnet (jedoch nicht vollständig, weil die Vorverriegelungsmöglichkeit gegeben sein muss), wie insbesondere Fig. 27 zu entnehmen ist. Fig. 25 zeigt einen Schnitt entlang der Linie XXV-XXV aus Fig. 24. Da sich die Halteblöcke in Bezug auf eine Erstreckungsrichtung der Spindeln auf derselben Höhe befinden, ist hier die Ansicht der beiden Halteblöcke 720, 720' identisch. Gut sichtbar sind insbesondere Federn 710, 710' und deren Enden 730a, 730a', 730b, 730b' (s. auch Fig. 22). Fig. 26 zeigt die Pumpeneinheit in einer Draufsicht, wobei die Oberseite des Ventildeckels mit Unterbrecherfahne an dem Steg 273 zu erkennen ist. Fig. 28 zeigt die Pumpeneinheit in einer Seitenansicht, wobei der Druckschlauch 105 deutlich wird.

Mit dem Verfahren der- Halteblöcke 720, 720' von der Referenzposition in die Einsetzposition, wie sie mit den Fig. 22 bis 28 gezeigt ist, werden bereits die Ausrückhebel 501, 501' betätigt und die Achsbolzen 502, 502' werden geringfügig aufeinander zubewegt. Beim Einsetzen der Pumpeneinheit 200 durch den Bediener werden die zur Vorverriegelung positionierten Achsbolzen durch den Zylinderblock 211 (bzw. durch Seitenflächen des Zylinderblocks) wieder leicht auseinander geschoben, so dass sich die Pumpeneinheit 200 zwischen die Achsbolzen in die Aufnahmeeinrichtung 510 zwängt. Sobald die Seitenflächen des Zylinderblocks die auseinander geschobenen Achsbolzen 502, 502' wieder freigeben, werden diese jeweils an der Ober- und Unterseite des Ventildeckels hinter den Haltestegen 273, 273' durch ein erneutes aufeinander zubewegen (bedingt durch die Zugfedern 503, 503') positioniert und die Pumpeneinheit 200 ist vorverriegelt. In der Einsetzposition sind die Ausrückhebel, da sie nur den Vorverriegelungszustand definieren, noch nicht vollständig geschlossen.

Durch das aufgrund der Unterbrechung (z. B. Reflexion) des Lichtstrahls der Lichtschranke generierte Signal (nach dem Einsetzen der Pumpeneinheit 200 in die Halteeinrichtung 500) werden die beiden Kupplungssysteme bzw. Kupplungseinrichtungen 130, 130' betätigt und die Kolbenhalter 700, 700' werden über die Spindeln von der zurückgezogenen Einsetzposition in Richtung Halteeinrichtung 500 nach vorne in die Befestigungs- und Ankoppelposition verfahren. Während des Verfahrens in die Befestigungs- und Ankoppelposition schließen die Ausrückhebel, vollständig (wie oben beschrieben), so dass die Pumpeneinheit 200 fest in der Halteeinrichtung 500 aufgenommen ist.

Durch das Verfahren der Halteblöcke in die Befestigungs- und Ankoppelposition stoßen die Halteblöcke 720, 720' an die Kolben an. Hier wird der Zweck der oben beschriebenen Vorverriegelung ersichtlich. Wäre eine Vorverriegelung nicht erfolgt, würden die Halteblöcke die Pumpeneinheit 200 wieder aus der Halteeinrichtung 500 auswerfen.

Die Halteblöcke 720, 720' sind an den Spindeln 470, 470' angeordnet und weisen Einsetzöffnungen 770, 770' auf, in welche die Kolbenstangen 250, 250' mit ihren Koppelvorsprüngen 170, 170' in der Befestigungs- und Ankoppelposition eingesetzt werden können. An den Halteblöcken 720, 720' sind die Federn 710, 710' so befestigt, dass sie durch die Halteblöcke 720, 720' hindurch verlaufen und die Federenden 730a, 730a', 730b, 730b' an einer Unterseite der Halteblöcke, Richtung Grundplatte 651 weisend, austreten (vgl. insbesondere Fig. 25). Wie aus Fig. 22 ersichtlich, ist an den Halteblöcken 720, 720' an deren Unterseiten Richtung Grundplatte 651 weisend, jeweils ein Schlitten 622, 622' angeordnet, wobei die Schlitten jeweils über Schlittenachsen 623, 623' an einer Richtung Halteeinrichtung weisenden Vorderseite der Halteblöcke 720, 720' befestigt sind. Die Schlitten sind über die Halteblöcke 720, 720' mit und über die Schlittenachsen 623, 623' relativ zu den Halteblöcken verfahrbar. Das heißt, Halteblöcke und Schlitten sind relativ zueinander verfahrbar.

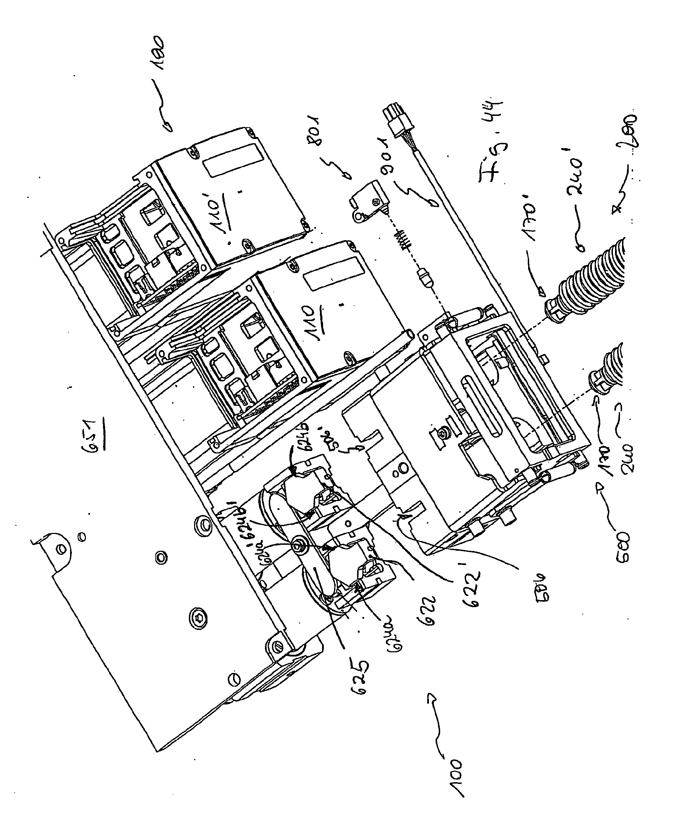

Durch das weitere nach vorne Verfahren der Kolbenhalter und damit der Halteblöcke in die Befestigungs und Ankoppelposition werden die Schlitten 622, 622' mit den Halteblöcken 720, 720' mitgenommen und stoßen schließlich an einer hinteren, in Richtung Kolbenhalter 700, 700' weisenden Seite der Halteeinrichtung 500 bzw. der Aufnahmeeinrichtung 510 an. Fig. 44 zeigt die Pumpenbetätigungseinrichtung 100 von unten. Hier sind auch beide Schlitten 622, 622' zu erkennen. Die Aufnahmeeinrichtung 510 ist an der hinteren Seite derart ausgebildet, dass sie die Schlitten zumindest teilweise aufnehmen kann. So sind beispielsweise Ausnehmungen 506, 506' vorgesehen, die einerseits die Schlittenachsen 623, 623' aufnehmen, aber auch ein hinteres Ende der Schlitten selbst. Grundsätzlich reicht es aus, die Ausnehmungen so zu gestalten, dass die Schlittenachsen eingeschoben werden können, damit sich die Schlitten gegen die hintere Seite der Aufnahmeeinrichtung abstützen können.

Wie insbesondere Fig. 44 zu entnehmen ist, sind die Schlitten 622, 622' derart ausgebildet, dass sie fluchtend in Spindelrichtung von einem sich verbreiternden Bereich in einen maximal breiten Bereich und von diesem wieder in einen sich verjüngenden Bereich übergehen (ähnlich einer Rautenform oder besser: Benzolringform). Die Schlitten weisen demgemäß Spreizflächen 624a, 624a', 624b, 624b' auf. Im noch entkoppelten Zustand (Kolben 250, 250' sind nicht an die Kolbenhalter 700, 700' angekoppelt) greifen jeweils die maximal breiten Bereiche mit ihren Spreizflächen 624a, 624a' bzw. 624b, 624b' zwischen die Federenden 730a, 730a' bzw. 730b, 730b' der Federn 710, 710' ein und halten die Federn gespreizt und damit "offen". Die Kolbenhalter 700, 700' werden durch die oben beschriebene Verfahrbewegung über die Spindeln derart auf die Kolben 250, 250' der vorverriegelten Pumpeneinheit 200 zubewegt, dass die Kolben 250, 250' in die Einsetzöffnungen 770, 770' eingeschoben werden. Gleichzeitig bzw. nach dem Aufnehmen der Kolben in den Halteblöcken stützen sich die Schlitten gegen die hintere Seite der Aufnahmeeinrichtung 510 bzw. Halteeinrichtung 500 ab, so dass die über den maximal breiten Bereichen der Schlitten geklemmten Federenden über die Spreizflächen auf den sich verjüngenden Bereich der Schlitten rutschen und so hinter den Koppelvorsprüngen 170, 170' der Kolben 250, 250' zusammenschnappen. Zum leichteren Einsetzen sind die Koppelvorsprünge 170, 170' an ihren Enden konisch ausgebildet. Nach dem Einsetzen der Koppelvorsprünge 170, 170' in die Kolbenhalter 700, 700' sind die Kolbenstangen 250, 250' schub- und zugfest mit den Kolbenhaltern 700, 700' bzw. mit den Halteblöcken 720, 720' verbunden. Zu diesem Zeitpunkt sind auch die Ausrückhebel vollständig geschlossen, weil die Gleitlager der Öffnungsschieber nicht auf den oder nur in unteren Bereichen der Öffnungsrampen aufliegen.

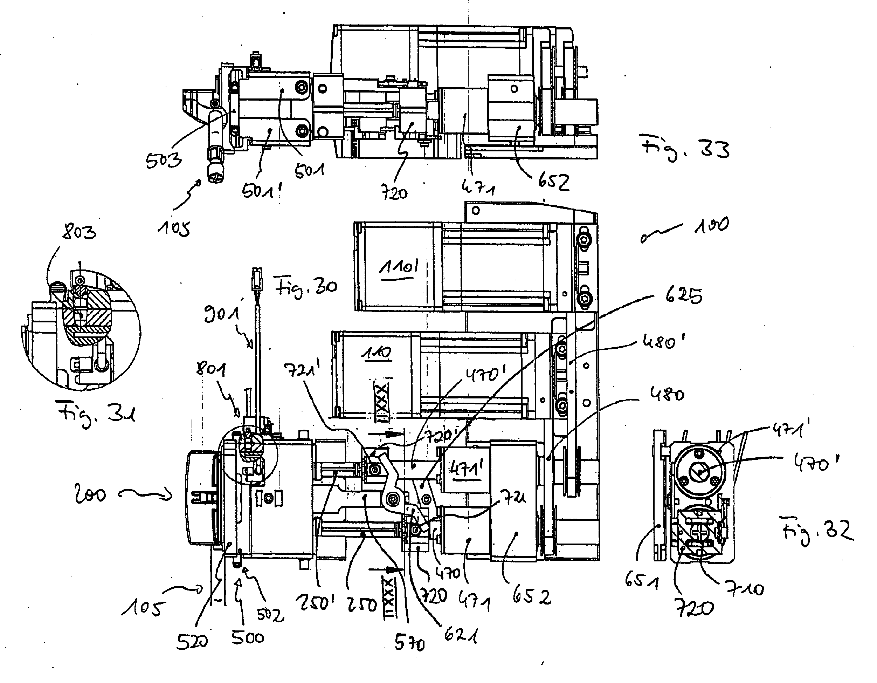

Die nun vollverriegelte Pumpeneinheit 200 (Ausrückhebel geschlossen, Kolben angekoppelt) mit den angekoppelten Kolben 250, 250' ist nun für den Betrieb bereit. Der Pumpenbetrieb wird über die Fig. 29 bis 33 verdeutlicht. Wie bereits oben näher beschrieben, erfolgt der Pumpenbetrieb durch alternierende Bewegung der Spindeln 470, 470' und damit der Kolben 250, 250'. Kolben und Spindeln 470, 470' sind in Fig. 30 ersichtlich. Hier sind die Faltenbalge nicht gezeigt.

Fig. 29 zeigt die medizinische Pumpe in ihrer Gesamtheit, nämlich die Pumpenbetätigungseinrichtung 100 mit eingesetzter Pumpeneinheit 200.

Aus Fig. 30 ist ebenfalls zu entnehmen, dass die Wippe 621 im alternierenden Betrieb der Pumpe wippenmäßig bewegt wird und daher den Öffnungsschieber 570 nicht betätigt. Der Öffnungsschieber 570 verbleibt also in der Position, bei der die Ausrückhebel 501, 501' vollständig geschlossen sind.

Fig. 32 zeigt einen Schnitt entlang der Linie XXXII-XXXII aus Fig. 30. Hier ist einerseits die Spindelaufnahme 471' mit Spindel 470' sichtbar, andererseits ist der Halteblock 720 im Schnitt gezeigt. Aus dem Schnitt wird deutlich, dass sich die Kolbenhalter nicht auf derselben Höhe in Bezug auf eine Erstreckungsrichtung der Spindeln befinden.

Fig. 31 zeigt einen Ausschnitt aus Fig. 30, nämlich den betätigten Mikroschalter 801, wobei der Betätigungsstift 801 gedrückt ist.

Fig. 33 zeigt die Anordnung gemäß Fig. 30 in einer Seitenansicht. Dabei wird deutlich, dass die Ausrückhebel 501, 501' im Pumpenbetrieb vollständig geschlossen sind.

Nachfolgend wird anhand der Fig. 34 bis 36 das Entnehmen der Pumpeneinheit 200 aus der Pumpenbetätigungseinrichtung 100 beschrieben.

Zum Entnehmen muss von einem Bediener ein entsprechendes Signal an die Motorsteuerung 150 zugeführt werden. Dies geschieht über das Loslassen bzw. die Betätigung des (nicht gezeigten) Fuß- oder Handschalters und dem Betätigen z. B. der Eject-Taste. Sobald der Hand- oder Fußschalter losgelassen wird, bleiben die Spindeln und damit die Kolben in der momentanen Position stehen, d. h. sie reagieren sofort auf das Abschaltsignal. Nur so lässt sich vermeiden, dass beispielsweise noch weiter ein Fluid über den Applikator an die entsprechende Stelle zugeführt wird.

Über das weitere Signal, z. B. dem Betätigen eines weiteren Schalters (Eject-Taste), wird ein Entriegelungsvorgang durch die Motorsteuerung 150 ausgelöst. Hier sei angemerkt, dass in der Praxis üblicherweise eine Art Touchscreen verwendet wird (wie bereits oben dargelegt). Unter dem Betätigen eines Schalters ist daher auch das Berühren eines entsprechenden Bereichs auf dem Bildschirm zu verstehen.

Die Motoren fahren die Spindeln und damit die Halteblöcke 720, 720' mit den noch angekoppelten Kolben nach hinten in die Löse- und Entkoppelposition (im Prinzip ist dies die Referenzposition). Die Schlitten 622, 622' stoßen in diesem Falle, sobald die Halteblöcke die hintere Position erreicht haben, an einer weiteren Wippe 625 an, die an einem zwischen den Spindelaufnahmen 471, 471' ausgebildeten, mit der Grundplatte verbundenen Steg angeordnet und insbesondere in Fig. 44 erkennbar ist. Diese Wippe, die sich im "normalen" Pumpenbetrieb ebenfalls mit den Kolben hin und her bewegt (um die Schlitten nicht zu betätigen), dient als weiterer Anschlag für die Schlitten 622, 622'. Die Schlitten stoßen an der Wippe an (dadurch, dass beide Schlitten an der Wippe 624 anstoßen, kann diese nicht ausweichen) und werden über die Schlittenachse 623 in Richtung der Kolben verschoben, so dass sich die Schlitten mit ihren Breitseiten wieder zwischen die Federenden 730a, 730a', 730b, 730b' schieben und die Federn 710, 710' spreizen können. Im Prinzip verschieben sich Halteblock und Schlitten bei Kontakt mit der Wippe relativ zueinander (werden voneinander bewegt), je nach Position der einzelnen Halteblöcke. Damit werden die Koppelvorsprünge 170, 170' der Kolben 250, 250' freigeben. Durch das wippenmäßige Mitbewegen der Wippe 625 kann diese während des normalen Pumpenbetriebs nicht als Anschlag für die Schlitten fungieren. Damit kann auch kein Eingreifen der Schlitten in die Federenden und ein Entriegeln der Kolben erfolgen.

Gleichzeitig mit dem Verfahren nach hinten in Richtung der Spindelaufnahmen, also in die Löse- und Entkoppelposition öffnen die Ausrückhebel (die Halteblöcke nehmen die Wippe 621 und damit den Öffnungsschieber 570 mit nach hinten). Allerdings ist der Mechanismus derart ausgelegt, dass ein vollständiges Öffnen der Ausrückhebel erst dann erfolgt, wenn die Kolben 250, 250' bereits aus den Halteblöcken 720, 720' entkoppelt sind. Ein Vorverriegelungsstadium ist hier nicht mehr vorgesehen. Die hier vorgegebene Reihenfolge der Entkopplung und Entriegelung (erst Entkoppeln der Kolben, dann Entriegeln der Halteeinrichtung) ist vorzugsweise deshalb vorgesehen, damit der Bediener die Pumpeneinheit erst dann aktiv aus der Halteeinrichtung entnehmen kann, wenn die Kolben entkoppelt sind. Andernfalls würde der Bediener ggf. an der in der geöffneten Halteeiririchtung befindlichen Pumpeneinheit ziehen, wobei ein Entkoppeln der Kolben noch nicht erfolgt ist. Dies würde ggf. zu einer Beschädigung des Pumpensystems führen.

Nun kann die Pumpeneinheit 200 durch die Bedienperson unproblematisch aus der Pumpenbetätigungseinrichtung 100 entfernt werden. Sobald die Pumpeneinheit tatsächlich entfernt wird, wird der Betätigungsstift 803 des Mikroschalters 801 losgelassen (der Mikrotaster wird deaktiviert), weil der Zylinderblock 211 (bzw. die Seitenwand) nicht mehr auf den Betätigungsstift drückt. Das somit generierte Signal, das zweite Detektionssignal D', wird an die Motorsteuerung 150 übermittelt, so dass die Motoren die Kolbenhalter 700, 700' bzw. die Halteblöcke 720, 720' in die Position verfahren, welche es ermöglicht, eine neue Pumpeneinheit einzusetzen (Einsetzposition). Damit wäre die Pumpenbetätigungseinrichtung 100 zum Einsetzen einer neuen Pumpeneinheit 200 bereit. In dieser Einsetzposition befindet sich die Pumpenbetätigungseinrichtung in dem Ruhezustand.

Fig. 34 zeigt die noch an die Pumpenbetätigungseinrichtung 100 angekoppelte Pumpeneinheit 100 von oben. Die Halteblöcke 720, 720' sind in der zurückgezogenen Position, der Öffnungsschieber 570 ist mit der Wippe 621 ebenfalls nach hinten gezogen, so dass, wie aus Fig. 36 ersichtlich, die Ausrückhebel 501, 501' vollständig zum Entnehmen der Pumpeneinheit 200 geöffnet sind.

Fig. 35 zeigt einen Schnitt entlang der Linie XXXV-XXXV aus Fig. 34. Wie aus dem Schnitt ersichtlich, befinden sich die Halteblöcke zum Entkoppeln in Bezug auf die Erstreckungsrichtung der Kolben auf derselben Höhe.

Fig. 37 zeigt die Pumpenbetätigungseinrichtung 100, wobei sich die Kolbenhalter 700, 700' mittels der Motoren 110, 110' in der Referenzposition befinden. Auch wenn Einsetzposition und Referenzposition gemäß der Abbildungen fast identisch erscheinen, so sei doch darauf hingewiesen, dass die Halteblöcke in den jeweiligen Positionen unterschiedlich positioniert sind (s. o.).

Fig. 38 zeigt eine etwas vergrößerte Ansicht der Pumpenbetätigungseinrichtung mit angekoppelter Pumpeneinheit, wie es bereits mit Fig. 24 dargestellt ist. Fig. 39 zeigt einen Schnitt entlang der Linie XXXIX-XXXIX aus Fig. 38. Dabei ist insbesondere die Führung 571 der Öffnungsschieberführung sichtbar. Der Öffnungsschieber 570 befindet sich mit der Wippe 621 über die Halteblöcke 720, 720' in der zurückgezogenen Einsetzposition. Insofern befinden sich die Gleitlager 573, 573' auf der höchsten Ebene der Öffnungsrampen 550, 550' und die Ausrückhebel 501, 501' sind geöffnet.

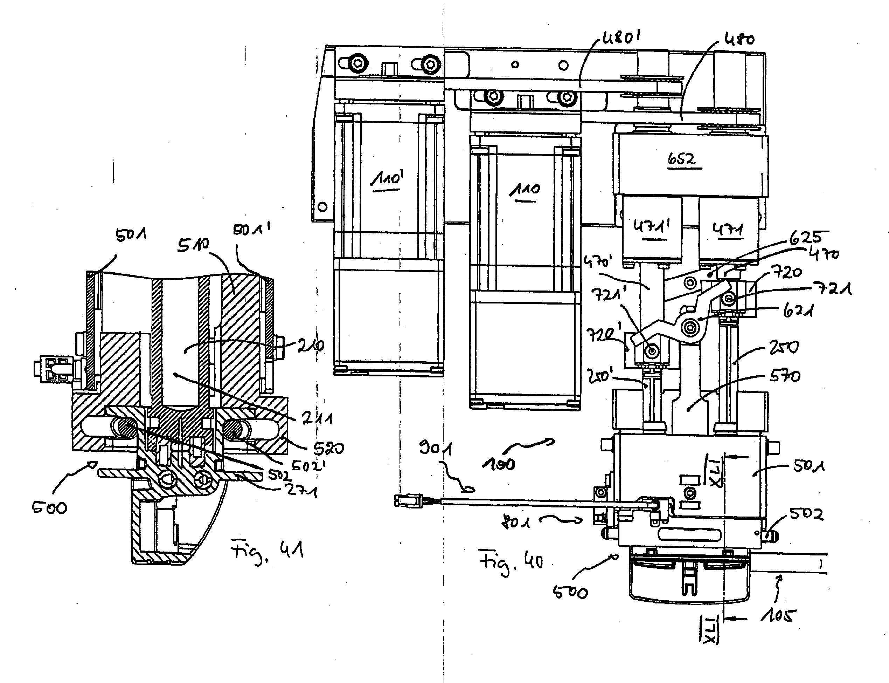

Fig. 40 zeigt eine etwas vergrößerte Ansicht der Pumpenbetätigungseinrichtung mit angekoppelter Pumpeneinheit gemäß Fig. 30. Fig. 41 zeigt einen Schnitt entlang der Linie XLI-XLI aus Fig. 40. Dabei ist, bei eingeschobener Pumpeneinheit 200 in die Halteeinrichtung 500, der Schnitt durch einen Zylinder 210 als Bestandteil des Zylinderblocks 211 gezeigt. Die Achsbolzen 502, 502' sind maximal zueinander zusammengeführt, da die Ausrückhebel geschlossen sind.

Sowohl die Lichtschranke, als auch der Mikroschalter (oder -taster) generieren Signale, die der Motorsteuerung bzw. Steuerung 150 übermittelt werden. Beim Einsetzen der Pumpeneinheit in die Pumpenbetätigungseinrichtung ist es jedoch vorgesehen, dass insbesondere das aufgrund der Aktivierung der Lichtschranke generierte Signal über die Steuerung ausgewertet wird und im Wesentlichen nur dieses Signal anzeigt, dass eine zum Befestigen und Ankoppeln bereite Pumpeneinheit eingesetzt ist. Sobald die Pumpeneinheit derart in der Pumpenbetätigungseinrichtung eingesetzt ist, dass die Lichtschranke "betätigt", der Lichtstrahl unterbrochen bzw. reflektiert wird, ist eine vorbestimmte Lagebeziehung zwischen Pumpeneinheit und Pumpenbetätigungseinrichtung erreicht - es wird also angezeigt, dass eine Pumpeneinheit eingesetzt wurde - und das Signal, das erste Detektionssignal D, löst die Ansteuerung der Motoren zum Verfahren letztendlich der Halteblöcke über die Spindeln aus. Aufgrund dieses Signals werden die Befestigungs- und Ankoppelposition und ggf. die Stand-by-Position angefahren. Aus dieser Position heraus lässt sich dann der Pumpenbetrieb auslösen. Sobald der Pumpenbetrieb unterbrochen und das gewünschte Entnehmen der benutzten Pumpeneinheit angezeigt wird, werden die Halteblöcke in die Löse- und Entkoppelposition verfahren. Mit dem Entnehmen der Pumpeneinheit aus der Halteeinrichtung wird der Mikroschalter deaktiviert und das so generierte, an die Steuerung übermittelte Signal, das zweite Detektionssignal D', veranlasst die Steuerung, die Motoren derart anzusteuern, dass die Halteblöcke in die Einsetzposition verfahren werden. Eine neue Pumpeneinheit kann eingesetzt werden. Die Einsetzposition ist im Prinzip als ein Ruhezustand oder Bereitschaftszustand zu verstehen.

Durch die Sensoren, d. h. durch die Detektionsmittel wird also insbesondere das Befestigen und Ankoppeln der Pumpeneinheit an der Pumpenbetätigungseinrichtung und damit das Ausbilden eines Arbeitszustandes der medizinischen Pumpe selbsttätig durch das System ausgelöst. Die Positionierung der Pumpenbetätigungseinrichtung in der Einsetzposition kann in diesen Ausführungsformen nach einer vorhergehenden Anwendung ebenfalls selbsttätig durchgeführt werden, hier z. B. aufgrund der Deaktivierung des Mikroschalters bzw. -tasters.

Gegebenenfalls wäre es möglich, das Befestigen und Ankoppeln erst dann auszulösen, wenn mindestens zwei Detektionsmittel ein Signal an die Steuerung übermitteln, so dass diese die Motoren nur dann ansteuert, wenn beide Signale kommuniziert werden. In den oben gezeigten Ausführungsformen ist es jedoch primär vorgesehen, für das Befestigen und Ankoppeln nur das Lichtschrankensignal als Detektionssignal auszuwerten.

### Bezugszeichenliste

- E: Einwegteil
- B: Bedienungsperson
- D, D': Detektionssignal
- 5: Druckschlauch
- 6: Fluideinlass
- 7: Fluidauslass
- 8: Applikator
- 9: Vorratsbehälter
- 10: Pumpenbetätigungseinrichtung
- 11, 11': Motor
- 12, 12': Getriebe
- 13, 13': Kupplungssystem, Kupplungseinrichtungen
- 14: Klemmventil
- 15: Motorsteuerung
- 16, 16': Druckraum
- 17, 17': Koppelvorsprung
- 18: Feder
- 19: Kugel
- 20: Pumpeneinheit
- 21, 21': Zylinder
- 22, 22': Kolben
- 23, 23': Dichtung
- 24, 24': Rollmembran
- 25, 25': Kolbenstange
- 26, 26': Saugventil
- 27, 27': Druckventil
- 28: Kappe
- 29: Zylinderkopf
- 30: Stellmotor
- 31: Kraftmesser
- 32: Regler
- 33: Feder
- 34: Stößelstange
- 35: Druckregelventil
- 36: Ventil-Membran
- 37: Überwurfstutzen
- 38: Crimprohr
- 39: Innenrohr
- 45: Schnappzunge
- 46: Haltevorsprünge
- 47, 47': Spindel
- 48, 48': Zahnriemen
- 50: Halteeinrichtung
- 51, 51': Klauen
- 52, 52': Klauenhalter
- 53: Druckfeder
- 54: Halteblock
- 55, 55': Öffnungsrampe
- 56, 56': Zylinderaufnahme
- 57: Öffnungsschieber
- 58: Feder
- 59, 59': Befestigungsschraube
- 60, 60': Langloch
- 61: Öffnungszunge
- 62: Kipphebel
- 63: Kipphebellager
- 64, 64': Zungen
- 65: Rahmen
- 70: Kolbenhalter/Rasteinrichtungen
- 71, 71': Feder
- 72, 72': Halteblock
- 73, 73': Federenden
- 74: Spreizhebel
- 75: Lager
- 76, 76': Spreizflächen
- 77, 77': Einsetzöffnung
- 78, 78': Schwenkkanten
- 100: Pumpenbetätigungseinrichtung
- 105: Druckschlauch
- 110, 110': Motor
- 130, 130': Kupplungssystem, Kupplungseinrichtungen
- 150: Motorsteuerung, Steuerung
- 170, 170': Koppelvorsprung
- 200: Pumpeneinheit, Pumpeinheit
- 210, 210': Zylinder
- 211: Zylinderblock
- 240, 240': Faltenbalg
- 250, 250': Kolben, Kolbenstange
- 271: Ventildeckel
- 272: Unterbrechereinrichtung
- 273, 273': Haltesteg
- 470, 470': Spindel
- 471, 471': Spindelaufnahme
- 480, 480': Zahnriemen
- 500: Halteeinrichtung
- 501, 501': Ausrückhebel
- 502, 502': Achsbolzen
- 503, 503': Zugfeder
- 504, 504': Zylinderstift
- 505, 505': Elastisches Element
- 506, 506': Ausnehmung
- 510: Aufnahmeeinrichtung
- 520: Aufnahmerahmen
- 550, 550': Öffnungsrampe
- 560, 560': Zylinderaufnahme
- 570: Öffnungsschieber
- 571: Öffnungsschieberführung
- 572: Langlöcher der Führung
- 573, 573': Gleitlager
- 574, 574': Achsbolzen
- 621: Wippe
- 622, 622': Schlitten
- 623, 623': Schlittenachse
- 624a, 624a': Spreizflächen
- 624b, 624b': Spreizflächen
- 625: Wippe
- 651: Grundplatte
- 652: Lagerbock
- 700, 700': Kolbenhalter
- 710, 710': Feder
- 720, 720': Halteblock
- 721, 721': Stutzeneinrichtung
- 730a, 730a', 730b, 730b': Federenden
- 770, 770': Einsetzöffnung
- 801: Mikroschalter
- 802: Druckfeder
- 803: Betätigungsstift
- 901: Lichtschranke
- 902: Steckerelement

## Patentansprüche

1. Medizinische Pumpe, insbesondere für die Wasserstrahlchirurgie, umfassend
- eine Pumpeneinheit (20, 200) mit zwei Kolben (22, 22', 25, 25', 250, 250') zum Verschieben der Kolben (22, 22', 25, 25', 250, 250') in zugeordneten Zylindern (21, 21', 210, 210');
- eine Pumpenbetätigungseinrichtung (10, 100) zum alternierenden Verschieben der Kolben (22, 22', 25, 25', 250, 250'), umfassend
- eine öffen- und schließbare Halteeinrichtung (50, 500) zum Befestigen und Lösen der Pumpeneinheit (20, 200) an der oder von der Pumpenbetätigungseinrichtung (10, 100);
- öffen- und schließbare Kupplungseinrichtungen (13, 13', 130, 130') zum Ankoppeln oder Entkoppeln der Kolben (22, 22', 25, 25', 250, 250') an die oder von der Pumpenbetätigungseinrichtung (10, 100);
- mindestens eine Antriebseinrichtung (11, 11', 12, 12', 110, 110', 120, 120') und eine Steuerung (15, 150) zum Betätigen der Pumpeneinheit (20, 200) durch alternierendes Verschieben der Kolben (22, 22', 25, 25', 250, 250'), wobei die Antriebseinrichtung (11, 11', 12, 12', 110, 110', 120, 120') zum Betätigen, insbesondere zum Öffnen und/oder Schließen, der Halteeinrichtung (50, 500) und/oder der Kupplungseinrichtungen (13, 13', 130, 130') derart steuerbar ausgebildet ist, dass ein Arbeitszustand, in dem die alternierende Kolbenbewegung stattfindet, oder ein Ruhezustand, in dem eine Pumpeneinheit in die Pumpenbetätigungseinrichtung eingesetzt werden kann, gebildet wird, und wobei die Antriebseinrichtung mit zwei Motoren (11, 11', 110, 110') oder einem Motor mit Getriebe derart steuerbar ausgebildet ist, dass die Kolben (22, 22', 25, 25', 250, 250') in einer vom alternierenden Betrieb abweichenden Weise zum Zweck des Öffnens oder Schließens der Halteeinrichtung (50, 500) und/oder der Kupplungseinrichtungen (13, 13', 130, 130') betrieben werden,
**gekennzeichnet durch**
- ein Detektionsmittel (801, 901), das mit einem Eingang der Steuerung kommunikativ verbunden und derart ausgebildet ist, dass es einen eine vorbestimmte Lagebeziehung zwischen Pumpeneinheit (20, 200) und Pumpenbetätigungseinrichtung (10, 100) definierenden Einsetzzustand detektiert und ein erstes, die Anwesenheit der Pumpeneinheit in der Pumpenbetätigungseinrichtung bezeichnendes Detektionssignal (D) an die Steuerung (15, 150) zum Steuern der Antriebseinrichtung (11, 11', 12, 12', 110, 110', 120, 120') übermittelt, und wobei das Detektionsmittel (801, 901) derart ausgebildet ist, dass es ein zweites, die Nicht-Anwesenheit der Pumpeneinheit in der Pumpenbetätigungseinrichtung bezeichnendes Detektionssignal (D') an die Steuerung (15, 150) zum Steuern der Antriebseinrichtung (11, 11', 12, 12', 110, 110', 120, 120') übermittelt,
wobei die Steuerung (15, 150) zur Verarbeitung des ersten Detektionssignals (D) derart ausgebildet ist, dass sie die Antriebseinrichtung (11, 11', 12, 12', 110, 110', 120, 120') zur Bildung des Arbeitszustandes steuert, und
wobei die Steuerung (15, 150) zur Verarbeitung des zweiten Detektionssignals (D') derart ausgebildet ist, dass sie die Antriebseinrichtung (11, 11', 12, 12', 110, 110', 120, 120') zur Bildung des Ruhezustandes steuert.

2. Medizinische Pumpe nach Anspruch 1,
**dadurch gekennzeichnet, dass**
mindestens ein Eingabeelement vorgesehen ist, das mit einem Eingang der Steuerung kommunikativ verbunden ist und ein Signal an die Steuerung übermittelt, wobei die Steuerung zur Verarbeitung des Signals derart ausgebildet ist, dass durch die Halteeinrichtung (50, 500) und/oder die Kupplungseinrichtungen (13, 13', 130, 130') eine Löse- und Entkoppelposition eingenommen wird.

3. Medizinische Pumpe nach einem der vorhergehenden Ansprüche,
**dadurch gekennzeichnet, dass**
das Detektionsmittel eine Lichtschranke (901) aufweist, wobei an der Pumpeneinheit (200) eine Unterbrechereinrichtung (272) zum Unterbrechen eines Lichtstrahles der Lichtschranke vorgesehen ist und wobei die Lichtschranke das erste Detektionssignal (D) bereitstellt.

4. Medizinische Pumpe nach Anspruch 3,
**dadurch gekennzeichnet, dass**
die Lichtschranke als eine Gabellichtschranke ausgebildet ist.

5. Medizinische Pumpe nach Anspruch 3 oder 4,
**dadurch gekennzeichnet, dass**
die Lichtschranke derart ausgebildet ist, dass sie auf Reflexion des von einem Sender der Lichtschranke ausgesendeten Lichtes anspricht.

6. Medizinische Pumpe nach einem der Ansprüche 3 bis 5,
**dadurch gekennzeichnet, dass**
das Detektionsmittel eine Taster- oder Schaltereinrichtung (801) aufweist, wobei die Taster- oder Schaltereinrichtung das zweite Detektionssignal (D')bereitstellt.

7. Medizinische Pumpe nach Anspruch 6,
**dadurch gekennzeichnet, dass**
die Tastereinrichtung- oder Schaltereinrichtung (801) als ein Mikroschalter oder - taster ausgebildet ist.

8. Medizinische Pumpe nach einem der vorhergehenden Ansprüche,
**dadurch gekennzeichnet, dass**
die Pumpenbetätigungseinrichtung (10, 100) erste Schnappverbindungseinrichtungen (51, 51', 72, 72', 71, 71', 502, 502', 720, 720' 710, 710') und die Pumpeneinheit zweite Schnappverbindungseinrichtungen (46, 17, 17', 273, 273', 170, 170') umfassen, so dass das Befestigen oder Lösen der Pumpeneinheit (20, 200) an der oder von der Pumpenbetätigungseinrichtung (10, 100) und/oder das Ankoppeln oder Entkoppeln der Kolben (22, 22', 25, 25', 250, 250' an die oder von der Pumpenbetätigungseinrichtung (10, 100) durch Ineinandergreifen oder Lösen der jeweiligen ersten und zweiten Schnappverbindungseinrichtungen erfolgt.

9. Medizinische Pumpe nach einem der vorhergehenden Ansprüche, insbesondere nach Anspruch 8,
**dadurch gekennzeichnet, dass**
die ersten Schnappverbindungseinrichtungen der Pumpenbetätigungseinrichtung (100) als Achsbolzen (502, 502') der Halteeinrichtung (500) und als Halteblöcke (720, 720') und zugehörige Federn (710, 710') der Kupplungseinrichtungen (130, 130') ausgebildet sind.

10. Medizinische Pumpe nach Anspruch 8 oder 9,
**dadurch gekennzeichnet, dass**
die zweiten Schnappverbindungseinrichtungen der Pumpeneinheit (200) als Haltestege (273, 273') an einem Ventildeckel (271) und als Koppelvorsprünge (170, 170') an Kolbenenden ausgebildet sind, wobei die Achsbolzen (502, 502') mit den Haltestegen (273, 273') und die Halteblöcke (720, 720') und zugehörigen Federn (710, 710') mit den Koppelvorsprüngen (170, 170') zusammenwirken, wenn Halteeinrichtung und Kupplungseinrichtungen geschlossen sind.

11. Medizinische Pumpe nach Anspruch 8,
**dadurch gekennzeichnet, dass**
die ersten Schnappverbindungseinrichtungen als Klauen (51, 51') an der Halteeinrichtung (50) und als Halteblöcke (72, 72') und zugehörige Federn (71, 71') der Kupplungseinrichtungen (13, 13') ausgebildet sind.

12. Medizinische Pumpe nach Anspruch 8 oder 11,
**dadurch gekennzeichnet, dass**
die zweiten Schnappverbindungseinrichtungen als Haltevorsprünge (46) an einem Zylinderkopf (29) und als Koppelvorsprünge (17, 17') an Kolbenenden ausgebildet sind, wobei die Klauen (51, 51') mit den Haltevorsprüngen (46) und die Halteblöcke (72, 72') und zugehörigen Federn (71, 71') mit den Koppelvorsprünge (17, 17') zusammenwirken, wenn Halteeinrichtung und Kupplungseinrichtungen geschlossen sind.

13. Medizinische Pumpe nach Ansprüche 8 bis 12,
**dadurch gekennzeichnet, dass**
die Steuerung (15, 150) derart ausgebildet ist, dass das Schließen der Halteeinrichtung (50, 500) und/oder der Kupplungseinrichtungen (13, 13', 130, 130') durch ein mittels der Antriebseinrichtung (11, 11', 12, 12', 110, 110', 120, 120') durchgeführtes selbsttätiges Einschnappen der jeweiligen ersten und zweiten Schnappverbindungseinrichtungen und das Öffnen durch ein mittels der Antriebseinrichtung (11, 11', 12, 12', 110, 110', 120, 120') durchgeführtes selbsttätiges Öffnen der Schnappverbindungseinrichtungen durchführbar ist.

14. Medizinische Pumpe nach einem der vorhergehenden Ansprüche,
**dadurch gekennzeichnet, dass**
die Antriebseinrichtung (11, 11', 12, 12', 110, 110', 120, 120') einen Linearantrieb mit mindestens einer Spindel (47, 470) und mindestens einem Motor (11, 110) zum steuerbaren Antrieb der Spindel (47, 470) umfasst.

15. Medizinische Pumpe nach einem der vorhergehenden Ansprüche,
**dadurch gekennzeichnet, dass**
die Motorsteuerung (15, 150') derart ausgebildet ist, dass die Kolben (22, 22', 25, 25', 250, 250') in konstanter Geschwindigkeit verschiebbar sind.

## Claims

1. Medical pump, in particular for water jet surgery, comprising
- a pump unit (20, 200) with two pistons (22, 22', 25, 25', 250, 250') for displacing the pistons (22, 22', 25, 25', 250, 250') in assigned cylinders (21, 21', 210, 210');
- a pump actuating device (10, 100) for alternating displacement of the pistons (22, 22', 25, 25', 250, 250'), comprising
- an openable and closable holding device (50, 500) for attaching and detaching the pump unit (20, 200) to or from the pump actuating device (10, 100);
- openable and closable coupling devices (13, 13', 130, 130') for coupling or uncoupling the pistons (22, 22', 25, 25', 250, 250') to or from the pump actuating device (10, 100);
- at least one drive device (11, 11', 12, 12', 110, 110', 120, 120') and a controller (15, 150) for actuating the pump unit (20, 200) by alternating displacement of the pistons (22, 22', 25, 25', 250, 250'), the drive device (11, 11', 12, 12', 110, 110', 120, 120') being controllably formed for actuating, in particular for opening and/or closing, the holding device (50, 500) and/or the coupling devices (13, 13', 130, 130') in such a way that a working state, in which the alternating piston movement takes place, or a resting state, in which a pump unit can be inserted into the pump actuating device, is formed, and the drive device with two motors (11, 11', 110, 110') or a motor with a gear mechanism being controllably formed in such a way that the pistons (22, 22', 25, 25', 250, 250') are operated in a way other than alternating operation for the purpose of opening or closing the holding device (50, 500) and/or the coupling devices (13, 13', 130, 130'),
**characterized by**
- a detection means (801, 901), which is communicatively connected to an input of the controller and is formed in such a way that it detects an insertion state, defining a predetermined positional relationship between the pump unit (20, 200) and the pump actuating device (10, 100), and transmits a first detection signal (D), denoting the presence of the pump unit in the pump actuating device, to the controller (15, 150) for controlling the drive device (11, 11', 12, 12', 110, 110', 120, 120'), and the detection means (801, 901) being formed in such a way that it transmits a second detection signal (D'), denoting the absence of the pump unit in the pump actuating device, to the controller (15, 150) for controlling the drive device (11, 11', 12, 12', 110, 110', 120, 120'),
the controller (15, 150) being formed for processing the first detection signal (D) in such a way that it controls the drive device (11, 11', 12, 12', 110, 110', 120, 120') to form the working state, and
the controller (15, 150) being formed for processing the second detection signal (D') in such a way that it controls the drive device (11, 11', 12, 12', 110, 110', 120, 120') to form the resting state.

2. Medical pump according to Claim 1, **characterized in that** at least one input element, which is communicatively connected to an input of the controller and transmits a signal to the controller, is provided, the controller being formed for processing the signal in such a way that a detaching and uncoupling position is assumed by the holding device (50, 500) and/or the coupling devices (13, 13', 130, 130').

3. Medical pump according to one of the preceding claims, **characterized in that** the detection means has a light barrier (901), an interrupter device (272) for interrupting a light beam of the light barrier being provided on the pump unit (200), and the light barrier providing the first detection signal (D).

4. Medical pump according to Claim 3, **characterized in that** the light barrier is formed as a fork light barrier.

5. Medical pump according to Claim 3 or 4, **characterized in that** the light barrier is formed in such a way that it responds to reflection of the light emitted by a transmitter of the light barrier.

6. Medical pump according to one of Claims 3 to 5, **characterized in that** the detection means has a pushbutton or switch device (801), the pushbutton or switch device providing the second detection signal (D').

7. Medical pump according to Claim 6, **characterized in that** the pushbutton device or switch device (801) is formed as a micro switch or micro pushbutton.

8. Medical pump according to one of the preceding claims, **characterized in that** the pump actuating device (10, 100) comprises first snap-in connecting devices (51, 51', 72, 72', 71, 71', 502, 502', 720, 720', 710, 710') and the pump unit comprises second snap-in connecting devices (46, 17, 17', 273, 273', 170, 170'), so that the attaching or detaching of the pump unit (20, 200) to or from the pump actuating device (10, 100) and/or the coupling or uncoupling of the pistons (22, 22', 25, 25', 250, 250') to or from the pump actuating device (10, 100) takes place by engaging or disengaging of the respective first and second snap-in connecting devices.

9. Medical pump according to one of the preceding claims, in particular according to Claim 8, **characterized in that** the first snap-in connecting devices of the pump actuating device (100) are formed as axle bolts (502, 502') of the holding device (500) and as holding blocks (720, 720') and associated springs (710, 710') of the coupling devices (130, 130').

10. Medical pump according to Claim 8 or 9, **characterized in that** the second snap-in connecting devices of the pump unit (200) are formed as holding ribs (273, 273') on a valve cover (271) and as coupling projections (170, 170') on piston ends, the axle bolts (502, 502') interacting with the holding ribs (273, 273') and the holding blocks (720, 720') and associated springs (710, 710') with the coupling projections (170, 170') when the holding device and the coupling devices are closed.

11. Medical pump according to Claim 8, **characterized in that** the first snap-in connecting devices are formed as claws (51, 51') on the holding device (50) and as holding blocks (72, 72') and associated springs (71, 71') of the coupling devices (13, 13').

12. Medical pump according to Claim 8 or 11, **characterized in that** the second snap-in connecting devices are formed as holding projections (46) on a cylinder head (29) and as coupling projections (17, 17') on piston ends, the claws (51, 51') interacting with the holding projections (46) and the holding blocks (72, 72') and associated springs (71, 71') interacting with the coupling projections (17, 17') when the holding device and the coupling devices are closed.

13. Medical pump according to Claims 8 to 12, **characterized in that** the controller (15, 150) is formed in such a way that the closing of the holding device (50, 500) and/or the coupling devices (13, 13', 130, 130') can be carried out by an automatic snapping in of the respective first and second snap-in connecting devices, carried out by means of the drive device (11, 11', 12, 12', 110, 110', 120, 120'), and the opening can be carried out by an automatic opening of the snap-in connecting devices, carried out by means of the drive device (11, 11', 12, 12', 110, 110', 120, 120').

14. Medical pump according to one of the preceding claims, **characterized in that** the drive device (11, 11', 12, 12', 110, 110', 120, 120') comprises a linear drive with at least one spindle (47, 470) and at least one motor (11, 110) for the controllable driving of the spindle (47, 470).

15. Medical pump according to one of the preceding claims, **characterized in that** the motor controller (15, 150') is formed in such a way that the pistons (22, 22' , 25, 25', 250, 250') are displaceable at a constant speed.

## Revendications

1. Pompe médicale, en particulier pour la chirurgie par jet d'eau, comprenant
- une unité de pompe (20, 200) comprenant deux pistons (22, 22', 25, 25', 250, 250') pour le déplacement des pistons (22, 22', 25, 25', 250, 250') dans des cylindres associés (21, 21', 210, 210') ;
- un dispositif d'actionnement de pompe (10, 100) pour le déplacement en alternance des pistons (22, 22', 25, 25', 250, 250'), comprenant
- un dispositif de retenue pouvant être ouvert et fermé (50, 500) pour la fixation de l'unité de pompe (20, 200) sur le dispositif d'actionnement de pompe (10, 100) et pour le desserrage de l'unité de pompe de celui-ci ;
- des dispositifs d'accouplement pouvant être ouverts et fermés (13, 13', 130, 130') pour l'accouplement des pistons (22, 22', 25, 25', 250, 250') au dispositif d'actionnement de pompe (10, 100) ou pour le désaccouplement des pistons de celui-ci ;
- au moins un dispositif d'entraînement (11, 11', 12, 12', 110, 110', 120, 120') et une commande (15, 150) pour l'actionnement de l'unité de pompe (20, 200) par déplacement en alternance des pistons (22, 22', 25, 25', 250, 250'), le dispositif d'entraînement (11, 11', 12, 12', 110, 110', 120, 120') étant réalisé de manière à pouvoir être commandé pour l'actionnement, en particulier pour l'ouverture et/ou la fermeture, du dispositif de retenue (50, 500) et/ou des dispositifs d'accouplement (13, 13', 130, 130') de telle sorte que l'on obtienne un état de travail, dans lequel a lieu le mouvement alternatif des pistons, ou un état de repos, dans lequel une unité de pompe peut être insérée dans le dispositif d'actionnement de pompe, et le dispositif d'entraînement étant réalisé de manière à pouvoir être commandé avec deux moteurs (11, 11', 110, 110') ou avec un moteur avec une transmission de telle sorte que les pistons (22, 22', 25, 25', 250, 250') fonctionnent de manière différente du mode alternatif afin d'ouvrir ou fermer le dispositif de retenue (50, 500) et/ou les dispositifs d'accouplement (13, 13', 130, 130'),
**caractérisée par**
- un moyen de détection (801, 901), qui est connecté par une liaison de communication à une entrée de la commande et qui est réalisé de telle sorte qu'il détecte un état d'insertion définissant une relation de position prédéterminée entre l'unité de pompe (20, 200) et le dispositif d'actionnement de pompe (10, 100) et qu'il communique un premier signal de détection (D) indiquant la présence de l'unité de pompe dans le dispositif d'actionnement de pompe à la commande (15, 150) pour commander le dispositif d'entraînement (11, 11', 12, 12', 110, 110', 120, 120'), et le moyen de détection (801, 901) étant réalisé de telle sorte qu'il communique un deuxième signal de détection (D') indiquant l'absence de l'unité de pompe dans le dispositif d'actionnement de pompe à la commande (15, 150) pour commander le dispositif d'entraînement (11, 11', 12, 12', 110, 110', 120, 120'),
la commande (15, 150) étant réalisée pour le traitement du premier signal de détection (D) de telle sorte qu'elle commande le dispositif d'entraînement (11, 11', 12, 12', 110, 110', 120, 120') pour qu'il établisse l'état de travail, et
la commande (15, 150) étant réalisée pour le traitement du deuxième signal de détection (D') de telle sorte qu'elle commande le dispositif d'entraînement (11, 11', 12, 12', 110, 110', 120, 120') pour qu'il établisse l'état de repos.

2. Pompe médicale selon la revendication 1, **caractérisée en ce**
**qu'**il est prévu au moins un élément d'entrée qui est connecté par une liaison de communication à une entrée de la commande et qui communique un signal à la commande, la commande étant réalisée pour le traitement du signal de telle sorte qu'une position de desserrage et de désaccouplement soit adoptée par le dispositif de retenue (50, 500) et/ou par les dispositifs d'accouplement (13, 13', 130, 130').

3. Pompe médicale selon l'une quelconque des revendications précédentes,
**caractérisée en ce que**
le moyen de détection présente une barrière lumineuse (901), un dispositif interrupteur (272) pour interrompre un faisceau lumineux de la barrière lumineuse étant prévu sur l'unité de pompe (200) et la barrière lumineuse fournissant le premier signal de détection (D).

4. Pompe médicale selon la revendication 3, **caractérisée en ce que**
la barrière lumineuse est réalisée sous forme de barrière lumineuse à fourche.

5. Pompe médicale selon la revendication 3 ou 4, **caractérisée en ce que**
la barrière lumineuse est réalisée de telle sorte qu'elle réagisse à la réflexion de la lumière émise par un émetteur de la barrière lumineuse.

6. Pompe médicale selon l'une quelconque des revendications 3 à 5,
**caractérisée en ce que**
le moyen de détection présente un dispositif palpeur ou commutateur (801), le dispositif palpeur ou commutateur fournissant le deuxième signal de détection (D').

7. Pompe médicale selon la revendication 6, **caractérisée en ce que**
le dispositif palpeur ou commutateur (801) est réalisé sous forme de mini-palpeur ou minirupteur.

8. Pompe médicale selon l'une quelconque des revendications précédentes,
**caractérisée en ce que**
le dispositif d'actionnement de pompe (10, 100) présente des premiers dispositifs de connexion par encliquetage (51, 51', 72, 72', 71, 71', 502, 502', 720, 720', 710, 710') et l'unité de pompe présente des deuxièmes dispositifs de connexion par encliquetage (46, 17, 17', 273, 273', 170, 170'), de telle sorte que la fixation de l'unité de pompe (20, 200) au dispositif d'actionnement de pompe (10, 100) ou le desserrage de l'unité de pompe de celui-ci et/ou l'accouplement des pistons (22, 22', 25, 25', 250, 250') au dispositif d'actionnement de pompe (10, 100) ou le désaccouplement des pistons de celui-ci s'effectuent par engagement les uns dans les autres, respectivement par desserrage les uns des autres, des premiers et deuxièmes dispositifs de connexion par encliquetage respectifs.

9. Pompe médicale selon l'une quelconque des revendications précédentes, en particulier selon la revendication 8,
**caractérisée en ce que**
les premiers dispositifs de connexion par encliquetage du dispositif d'actionnement de pompe (100) sont réalisés sous forme d'axes-pivots (502, 502') du dispositif de retenue (500) et sous forme de blocs de retenue (720, 720') et de ressorts associés (710, 710') des dispositifs d'accouplement (130, 130').

10. Pompe médicale selon la revendication 8 ou 9, **caractérisée en ce que**
les deuxièmes dispositifs de connexion par encliquetage de l'unité de pompe (200) sont réalisés sous forme de nervures de retenue (273, 273') sur un chapeau de soupape (271) et sous forme de saillies de couplage (170, 170') sur les extrémités des pistons, les axes-pivots (502, 502') coopèrant avec les nervures de retenue (273, 273') et les blocs de retenue (720, 720') et les ressorts associés (710, 710') coopèrant avec les saillies de couplage (170, 170') lorsque le dispositif de retenue et les dispositifs d'accouplement sont fermés.

11. Pompe médicale selon la revendication 8, **caractérisée en ce que**
les premiers dispositifs de connexion par encliquetage sont réalisés sous forme de griffes (51, 51') sur le dispositif de retenue (50) et sous forme de blocs de retenue (72, 72') et de ressorts associés (71, 71') des dispositifs d'accouplement (13, 13').

12. Pompe médicale selon la revendication 8 ou 11, **caractérisée en ce que**
les deuxièmes dispositifs de connexion par encliquetage sont réalisés sous forme de saillies de retenue (46) sur une culasse (29) et sous forme de saillies de couplage (17, 17') sur les extrémités des pistons, les griffes (51, 51') coopérant avec les saillies de retenue (46) et les blocs de retenue (72, 72') et les ressorts associés (71, 71') coopérant avec les saillies de couplage (17, 17'), lorsque le dispositif de retenue et les dispositifs d'accouplement sont fermés.

13. Pompe médicale selon les revendications 8 à 12, **caractérisée en ce que**
la commande (15, 150) est réalisée de telle sorte que la fermeture du dispositif de retenue (50, 500) et/ou des dispositifs d'accouplement (13, 13', 130, 130') puisse être effectuée par un encliquetage automatique effectué au moyen du dispositif d'entraînement (11, 11', 12, 12', 110, 110', 120, 120') de chacun des premiers et deuxièmes dispositifs de connexion par encliquetage et que l'ouverture puisse être effectuée par une ouverture automatique des dispositifs de connexion par encliquetage effectuée au moyen du dispositif d'entraînement (11, 11', 12, 12', 110, 110', 120, 120').

14. Pompe médicale selon l'une quelconque des revendications précédentes,
**caractérisée en ce que**
le dispositif d'entraînement (11, 11', 12, 12', 110, 100', 120, 120') comprend un entraînement linéaire avec au moins une broche (47, 470) et au moins un moteur (11, 110) pour l'entraînement commandable de la broche (47, 470).

15. Pompe médicale selon l'une quelconque des revendications précédentes,
**caractérisée en ce que**
la commande par moteur (15, 150') est réalisée de telle sorte que les pistons (22, 22', 25, 25', 250, 250') puissent être déplacés avec une vitesse constante.
